# EUROPÄISCHE PATENTSCHRIFT

(11) **EP 1 654 388 B1**
(45) Veröffentlichungstag und Bekanntmachung des Hinweises auf die Patenterteilung: **27.06.2007**
(21) Anmeldenummer: 04816095.6
(22) Anmeldetag: 13.08.2004
(51) Int. Cl.: C12Q 1/68

(54) **VERFAHREN ZUM NACHWEIS VON CYTOSIN-METHYLIERUNGEN IN DNA**
METHOD FOR THE DETECTION OF CYTOSINE METHYLATIONS IN DNA
PROCEDE DE DETECTION DE METHYLATION DE CYTOSINE DANS DE L'ADN

(30) Priorität: 15.08.2003 DE 10338308
(43) Veröffentlichungstag der Anmeldung: 10.05.2006
(73) Patentinhaber: Epigenomics AG, 10178 Berlin (DE)
(72) Erfinder: TETZNER, Reimo, 10439 Berlin (DE); DISTLER, Jürgen, 10825 Berlin (DE)
(74) Vertreter: Schubert, Klemens
(86) Internationale Anmeldenummer: PCT/DE2004/001837
(87) Internationale Veröffentlichungsnummer: WO 2005/024056

(56) Entgegenhaltungen:
- WO-A-99/66071
- US-A1- 2003 087 240
- US-B1- 6 331 393
- WHITCOMBE D ET AL: "Detection of PCR products using self-probing amplicons and fluorescence" NATURE BIOTECHNOLOGY, NATURE PUBLISHING, US, Bd. 17, Nr. 8, August 1999 (1999-08), Seiten 804-807, XP002226672 ISSN: 1087-0156
- THELWELL N ET AL: "Mode of action and application of Scorpion primers to mutation detection." NUCLEIC ACIDS RESEARCH. 1 OCT 2000, Bd. 28, Nr. 19, 1. Oktober 2000 (2000-10-01), Seiten 3752-3761, XP002247004 ISSN: 1362-4962
- WALKER N J: "Real-time and quantitative PCR: applications to mechanism-based toxicology." JOURNAL OF BIOCHEMICAL AND MOLECULAR TOXICOLOGY. 2001, Bd. 15, Nr. 3, 2001, Seiten 121-127, XP009043979 ISSN: 1095-6670
- COTTRELL S E ET AL: "SENSITIVE DETECTION OF DNA METHYLATION" ANNALS OF THE NEW YORK ACADEMY OF SCIENCES, NEW YORK ACADEMY OF SCIENCES, NEW YORK, NY, US, Bd. 983, 2003, Seiten 120-130, XP009043996 ISSN: 0077-8923
- LIU ZE-JUN ET AL: "Polymerase chain reaction-based methods of DNA methylation analysis." ANALYTICAL BIOCHEMISTRY, Bd. 317, Nr. 2, 15. Juni 2003 (2003-06-15), Seiten 259-265, XP002318907 ISSN: 0003-2697
- LO Y M DENNIS ET AL: "Quantitative analysis of aberrant p16 methylation using real-time quantitative methylation-specific polymerase chain reaction" CANCER RESEARCH, Bd. 59, Nr. 16, 15. August 1999 (1999-08-15), Seiten 3899-3903, XP009044313 ISSN: 0008-5472
- EADS C A ET AL: "MethyLight: a high-throughput assay to measure DNA methylation." NUCLEIC ACIDS RESEARCH. 15 APR 2000, Bd. 28, Nr. 8, 15. April 2000 (2000-04-15), Seiten E32-1-E32-8, XP002318908 ISSN: 1362-4962 in der Anmeldung erwähnt
- SOLINAS A ET AL: "Duplex Scorpion primers in SNP analysis and FRET applications." NUCLEIC ACIDS RESEARCH. 15 OCT 2001, Bd. 29, Nr. 20, 15. Oktober 2001 (2001-10-15), Seiten E96-1-E96-9, XP002318909 ISSN: 1362-4962
- PINERO DAVID ET AL: "Analytical challenges for the detection of genetic modifications in food and food products." AMERICAN BIOTECHNOLOGY LABORATORY, Bd. 21, Nr. 4, März 2003 (2003-03), Seiten 40-41, XP009044306 ISSN: 0749-3223
- MCKEEN CATHERINE M ET AL: "Synthesis of fluorophore and quencher monomers for use in scorpion primers and nucleic acid structural probes." ORGANIC & BIOMOLECULAR CHEMISTRY. 7 JUL 2003, Bd. 1, Nr. 13, 7. Juli 2003 (2003-07-07), Seiten 2267-2275, XP002318910 ISSN: 1477-0520
- LAIRD PETER W: "The power and the promise of DNA methylation markers." NATURE REVIEWS CANCER, Bd. 3, Nr. 4, April 2003 (2003-04), Seiten 253-266, XP009044309 ISSN: 1474-175X
- RAND K ET AL: "Conversion-specific detection of DNA methylation using real-time polymerase chain reaction (ConLight-MSP) to avoid false positives" METHODS : A COMPANION TO METHODS IN ENZYMOLOGY, ACADEMIC PRESS INC., NEW YORK, NY, US, Bd. 27, Nr. 2, Juni 2002 (2002-06), Seiten 114-120, XP002296074 ISSN: 1046-2023
- TRINH BINH N ET AL: "DNA methylation analysis by MethyLight technology" METHODS (ORLANDO), Bd. 25, Nr. 4, Dezember 2001 (2001-12), Seiten 456-462, XP002318911 ISSN: 1046-2023
- BRANSTEITTER R ET AL: "Activation-induced cytidine deaminase deaminates deoxycytidine on single-stranded DNA but requires the action of RNase" PROCEEDINGS OF THE NATIONAL ACADEMY OF SCIENCES OF USA, NATIONAL ACADEMY OF SCIENCE. WASHINGTON, US, Bd. 100, Nr. 7, 1. April 2003 (2003-04-01), Seiten 4102-4107, XP002302399 ISSN: 0027-8424
- PHAM P ET AL: "Processive AID-catalysed cytosine deamination on single-stranded DNA simulates somatic hypermutation" NATURE, MACMILLAN JOURNALS LTD. LONDON, GB, Bd. 424, Nr. 6944, 3. Juli 2003 (2003-07-03), Seiten 103-107, XP002302398 ISSN: 0028-0836
- DAHL C ET AL.: "DNA methylation analysis techniques" BIOGERONTOLOGY, Bd. 4, August 2003 (2003-08), Seiten 233-250,
- SAHA BK ET AL.: "Quantitation of HIV-1 by real-time PCR with a unique fluorogenic probe" JOURNAL OF VIROLOGICAL METHODS, Bd. 93, 2001, Seiten 33-42,

### Sequence listing

<110> Epigenomics AG
<120> Verfahren zum Nachweis von Cytosin-Methylierungen in DNA
<160> 16
<210> 1
   <211> 26
   <212> DNA
   <213> Artificial Sequence
<220>
   <223> chemically treated genomic DNA (Homo sapiens)
<400> 1
   gaagttgata gatgggtatt ttttga 26
<210> 2
   <211> 19
   <212> DNA
   <213> Artificial Sequence
<220>
   <223> chemically treated genomic DNA (Homo sapiens)
<400> 2
   cccccttcct aatcctcaa 19
<210> 3
   <211> 19
   <212> DNA
   <213> Artificial Sequence
<220>
   <223> chemically treated genomic DNA (Homo sapiens)
<400> 3
   gcggaatttg agaggcgta 19
<210> 4
   <211> 20
   <212> DNA
   <213> Artificial Sequence
<220>
   <223> chemically treated genomic DNA (Homo sapiens)
<400> 4
   gcgttgtgaa ttttggggag 20
<210> 5
   <211> 27
   <212> DNA
   <213> Artificial Sequence
<220>
   <223> chemically treated genomic DNA (Homo sapiens)
<400> 5
   taatcctcaa cacttccctc acaacct 27
<210> 6
   <211> 29
   <212> DNA
   <213> Artificial Sequence
<220>
   <223> chemically treated genomic DNA (Homo sapiens)
<400> 6
   ggcagcctag gtcgcgaggg aaggctgcc 29
<210> 7
   <211> 128
   <212> DNA
   <213> Artificial Sequence
<220>
   <223> chemically treated genomic DNA (Homo sapiens)
<400> 7
   gaagttgata gatgggtatt ttttgacggg gggtaggggc ggaatttgag aggcgtaagg      60
   cgttgtgaat tttggggagg ggggtagttt gtaggtcgcg agggaagcgt tgaggattag     120
   gaaggggg                                                              128
<210> 8
   <211> 19
   <212> DNA
   <213> Artificial Sequence
<220>
   <223> chemically treated genomic DNA (Homo sapiens)
<400> 8
   cccccttcct aatcctcaa 19
<210> 9
   <211> 74
   <212> DNA
   <213> Artificial Sequence
<220>
   <223> chemically treated genomic DNA (Homo sapiens)
<400> 9
   cgggattatt tttataaggt tcggaggtcg cgaggttttc gttggagttt cgtcgtcgta 60
   gttttcgtta ttag 74
<210> 10
   <211> 20
   <212> DNA
   <213> Artificial Sequence
<220>
   <223> chemically treated genomic DNA (Homo sapiens)
<400> 10
   gggattattt ttataaggtt 20
<210> 11
   <211> 22
   <212> DNA
   <213> Artificial Sequence
<220>
   <223> chemically treated genomic DNA (Homo sapiens)
<400> 11
   tactcactaa taacaaaact ac 22
<210> 12
   <211> 29
   <212> DNA
   <213> Artificial Sequence
<220>
   <223> chemically treated genomic DNA (Homo sapiens)
<400> 12
   ggcagccgtt ggagtttcgt cgggctgcc 29
<210> 13
   <211> 22
   <212> DNA
   <213> Artificial Sequence
<220>
   <223> chemically treated genomic DNA (Homo sapiens)
<400> 13
   tactcactaa taacaaaact ac 22
<210> 14
   <211> 34
   <212> DNA
   <213> Artificial Sequence
<220>
   <223> chemically treated genomic DNA (Homo sapiens)
<400> 14
   ctaataacaa aaactacaac aacaaaactc caac 34
<210> 15
   <211> 15
   <212> DNA
   <213> Artificial Sequence
<220>
   <223> chemically treated genomic DNA (Homo sapiens)
<400> 15
   gttggagttt cgtcg 15
<210> 16
   <211> 22
   <212> DNA
   <213> Artificial Sequence
<220>
   <223> chemically treated genomic DNA (Homo sapiens)
<400> 16
   tactcactaa taacaaaact ac 22

## Beschreibung

### Hintergrund der Erfindung

Die vorliegende Erfindung betrifft ein Verfahren zum Nachweis von 5-Methylcytosin in DNA. 5-Methylcytosin ist die häufigste kovalent modifizierte Base in der DNA eukaryontischer Zellen. Sie spielt eine wichtige biologische Rolle, u.a. bei der Transkriptionsregulation, beim genetischen Imprinting und in der Tumorgenese (zur Übersicht: Millar et al.: Five not four: History and significance of the fifth base. In: The Epigenome, S. Beck and A. Olek (eds.), Wiley-VCH Verlag Weinheim 2003, S. 3-20). Die Identifizierung von 5-Methylcytosin als Bestandteil genetischer Information ist daher von erheblichen Interesse. Ein Nachweis der Methylierung ist allerdings schwierig, da Cytosin und 5-Methylcytosin das gleiche Basenpaarungsverhalten aufweisen. Viele der herkömmlichen, auf Hybridisierung beruhenden Nachweisverfahren vermögen daher nicht zwischen Cytosin und Methylcytosin zu unterscheiden. Zudem geht die Methylierungsinformation bei einer PCR-Amplifikation vollständig verloren.

Die herkömmlichen Methoden zur Methylierungsanalyse arbeiten im wesentlichen nach zwei unterschiedlichen Prinzipien. Zum einen werden methylierungsspezifische Restriktionsenzyme benutzt, zum anderen erfolgt eine selektive chemische Umwandlung von nicht-methylierten Cytosinen in Uracil (sog.: Bisulfit-Behandlung, siehe etwa: DE 101 54 317 A1; DE 100 29 915 A1). Die enzymatisch oder chemisch vorbehandelte DNA wird dann meist amplifiziert und kann auf unterschiedliche Weise analysiert werden (zur Übersicht: WO 02/072880 S. 1 ff). Von großem Interesse sind dabei Verfahren, die in der Lage sind, Methylierung sensitiv und quantitativ zu detektieren. Dies gilt aufgrund der wichtigen Rolle der CytosinMethylierung in der Krebsentstehung insbesondere in Hinblick auf diagnostische Anwendungen. Von besonderer Bedeutung sind dabei Methoden, die es erlauben, abweichende Methylierungsmuster in Körperflüssigkeiten, etwa in Serum, nachzuweisen. Denn anders als die instabile RNA ist DNA oft in Körperflüssigkeiten anzutreffen. Bei destruktiven pathologischen Prozessen wie Krebserkrankungen ist die DNA-Konzentration im Blut sogar erhöht. Eine Krebsdiagnostik über eine Methylierungsanalyse von in Körperflüssigkeiten befindlicher Tumor-DNA ist also möglich und schon mehrfach beschrieben (siehe etwa: Palmisano et al.: Predicting lung cancer by detecting aberrant promoter methylation in sputum. Cancer Res. 2000 Nov 1;60(21) :5954-8). Ein Problem besteht dabei darin, dass sich in den Körperflüssigkeiten neben der DNA mit dem krankheitstypischen Methylierungsmuster eine große Menge an DNA der gleichen Sequenz aber eines anderen Methylierungsmusters befindet. Die Diagnoseverfahren müssen daher in der Lage sein, geringe Mengen besonders methylierter DNA vor einem starken Hintergrund an DNA derselben Sequenz aber eines anderen Methylierungsmusters nachzuweisen.

Die gängigen Verfahren zur sensitiven Detektion erfolgen über eine PCR-Amplifikation. Eine Methode ist dabei die sogenannte methylierungssensitive PCR ("MSP"; Herman et al.: Methylation-specific PCR: a novel PCR assay for methylation status of CpG islands. Proc Natl Acad Sci U S A. 1996 Sep 3;93(18):9821-6). Dabei werden Primer verwendet, die spezifisch nur an Positionen der Bisulfit-behandelten Sequenz binden, die vorher entweder methyliert (oder bei umgekehrtem Ansatz: unmethyliert) waren. Ein vergleichbar sensitives Verfahren ist die sogenannte "Heavy Methyl"-Methode. Dabei wird eine spezifische Amplifizierung nur der ursprünglich methylierten (bzw. unmethylierten) DNA durch Einsatz von methylierungsspezifischen Blocker-Oligomeren erreicht (zur Übersicht: WO 02/072880). Sowohl MSP wie Heavy Methyl sind als quantifizierbare Real-Time-Varianten anwendbar. Diese ermöglichen es, den Methylierungsstatus weniger Positionen direkt im Verlauf der PCR nachzuweisen, ohne dass eine nachfolgende Analyse der Produkte erforderlich wäre ("MethyLight" - WO00/70090; US 6,331,393). Eine Ausführungsform ist dabei das "Taqman"-Verfahren. Dieses verwendet Sondenmoleküle, die ein Fluoreszenzfarbstoff-Quencher-Paar tragen. Die Sonden hybridisieren sequenzspezifisch an die Amplifikate und werden im Zuge des nächsten Amplifikationszyklus durch die Exonuklease-Aktivität der Polymerase abgebaut. Durch die Trennung von Quencher und Farbstoff entsteht ein detektierbares Fluoreszenz-Signal (siehe etwa Eads et al.: MethyLight: a high-throughput assay to measure DNA methylation. Nucleic Acids Res. 2000 Apr 15;28(8):E32). Eine weitere MethyLight-Ausführungsform ist das sog. Lightcycler-Verfahren. Dabei werden zwei unterschiedliche Sonden eingesetzt, die in unmittelbarer Nähe zueinander an das Amplifikat hybridisieren, und die dann über Fluoreszenz-Resonanz-Energie-Transfer (FRET) ein detektierbares Signal erzeugen.

Die Anwendbarkeit dieser Real-Time-Verfahren auf die Methylierungsanalyse ist allerdings beschränkt. Dies gilt insbesondere in Bezug auf Spezifität, Sensitivität und Reaktionsgeschwindigkeit. Aufgrund der besonderen biologischen und medizinischen Bedeutung der CytosinMethylierung besteht aber ein starkes technisches Bedürfnis an der Entwicklung leistungsfähiger Methoden zur Methylierungsanalyse. Im folgenden ist ein solches Verfahren beschrieben. Hierin sind Sonde und Primer miteinander verbunden, so dass die Hybridisierung der Sonde an die Zielsequenz intramolekular erfolgen kann. Das erfindungsgemäße Verfahren erlaubt eine effektive und schnelle Detektion und ermöglicht somit eine sehr sensitive und sehr spezifische Methylierungsanalyse.

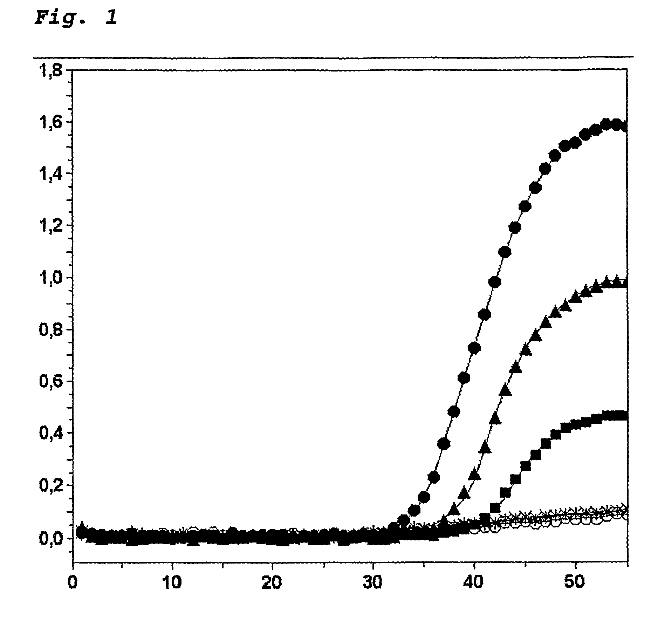

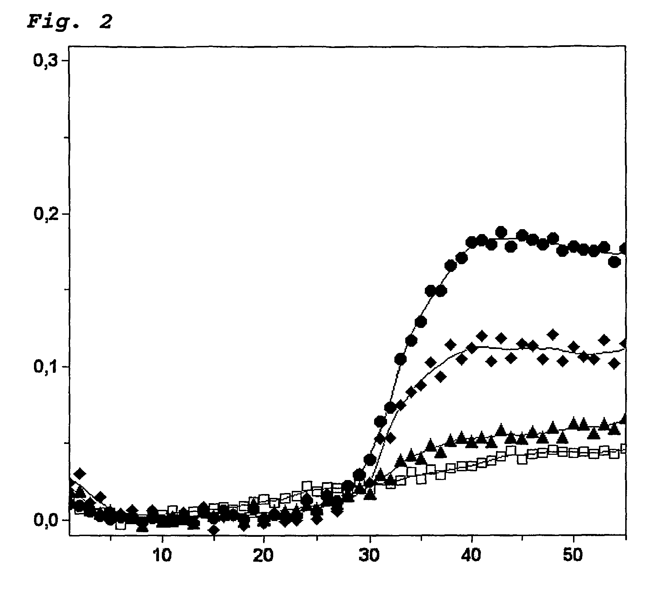

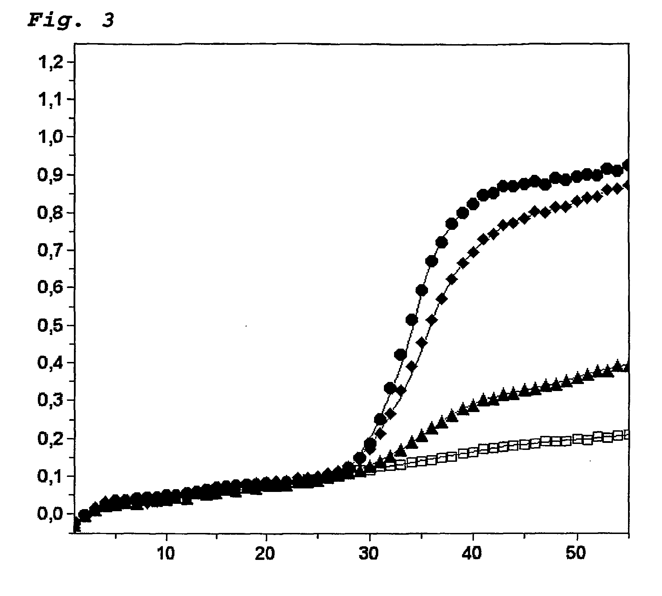

Eine zum erfindungsgemäßen Verfahren ähnliche Methode zur Mutationsanalyse ist bereits unter dem Namen "Scorpion" beschrieben (siehe etwa: Whitcombe et al.: Detection of PCR products using self-probing amplicons and fluorescence. Nat Biotechnol. 1999 Aug;17(8):804-7; Thelwell et al.: Mode of action and application of Scorpion primers to mutation detection. Nucleic Acids Res. 2000 Oct 1;28(19):3752-61; US 6,326,145; US 6,365,729; US 20030087240 A1). Das Scorpion-Verfahren ist in unterschiedlichen Ausführungsformen anwendbar. Allen Verfahren ist allerdings die intramolekulare Sondenbindung gemeinsam. In der sog. "Haarnadel-Schleifen"-Variante tragen die Scorpion-Primer am 5'-Ende eine spezifische Sondensequenz, die in einer besonderen Haarnadel-Schleifen-Konfiguration vorliegt. An den Enden der Sondensequenz befinden sich ein Fluoreszenzfarbstoff und ein Quencher, die durch die Haarnadelbildung in unmittelbare räumliche Nähe zueinander gelangen. Die Sonden- und die Primersequenz sind über einen Linker verbunden, der einen sog. PCR-Stopper trägt. Wird nach einer Amplifikationsrunde der entstandene Doppelstrang getrennt, so bindet die Sonde intramolekular an die verlängerte Primer-Sequenz desselben Stranges. Durch diese Hybridisierung wird die Haarnadel geöffnet, so dass Fluoreszenzfarbstoff und Quencher getrennt werden, und somit ein Signal detektiert werden kann. Der PCR-Stopper verhindern ein "Durchlesen" der Polymerase innerhalb der PCR und vermeidet so falschpositive Signale (Vgl.: Thelwell et al. 2000, a.a.o, insbesondere Abb. 1, S. 3753).

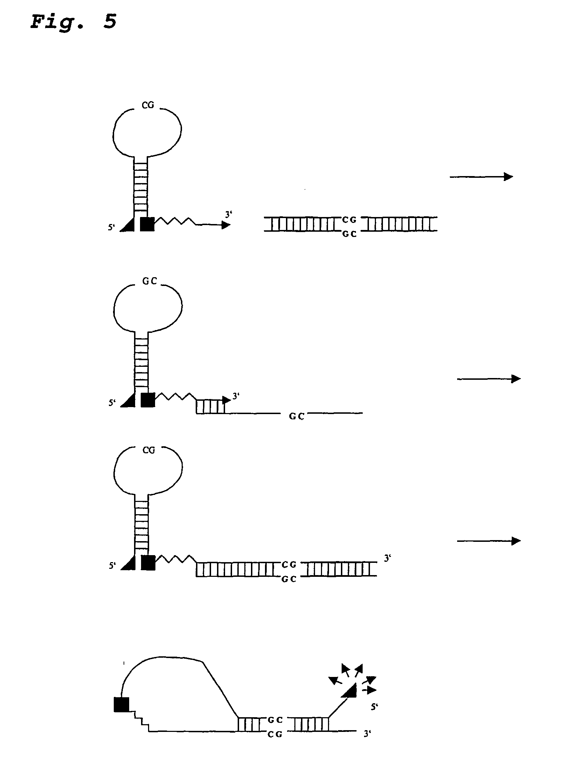

Eine andere Scorpion-Variante ist das sog. "Duplex"-Verfahren. Die Sondensequenz liegt hier nicht in einer Haarnadelstruktur vor, sondern bildet mit einem weiteren Oligonukleotid eine Duplex. Dabei ist an das 5'-Ende der Sondensequenz ein Fluoreszenzfarbstoff gebunden, während das andere Oligonuleotid einen Quencher am 3'-Ende trägt. Durch die Duplex-Bildung befinden sich Quencher und Farbstoff in unmittelbarer räumlicher Nähe. Werden nach einer Amplifikationsrunde die Doppelstränge getrennt, so bindet die Sonde intramolekular an die verlängerte Primer-Sequenz desselben Stranges. Fluoreszenzfarbstoff und Quencher werden getrennt, so dass ein Signal detektiert werden kann (Solinas et al.: Duplex Scorpion primers in SNP analysis and FRET applications. Nucleic Acids Res. 2001 Oct 15;29(20):E96). Es sind zudem auch Duplex-Varianten beschrieben, in denen die Sonde zwei Farbstoffe trägt, und bei denen die Signalbildung über einen Fluoreszenz-Resonanz-Energie-Transfer erfolgt (Vgl.: Solinas et al. 2001, a.a.o. insbesondere S. 7 f und S. 6 Abb. 5). Ein Vorteil der Duplex-Verfahren gegenüber dem oben beschriebenen Haarnadel-Verfahren besteht darin, dass in der aktivierten Form durch die vollständige Trennung von Quencher und Farbstoff ein stärkeres Fluoreszenz-Signal entsteht. Zudem sind Duplex-Scorpion-Primer einfacher zu synthetisieren und preiswerter als die entsprechenden Haarnadel-Primer (vgl.: Solinas et al. 2001, a.a.o. S.8 f) .

Weitere Scorpion-Varianten sind ausführlich im US-Patent 6326145 und in der US-Patentanmeldung 20030087240 beschrieben.

Die Scorpion-Verfahren haben mehrere Vorteile gegenüber den herkömmlichen Real-Time-PCR-Methoden. Dies gilt insbesondere in Hinblick auf die Reaktionsgeschwindigkeit. So hybridisieren die Scorpion-Sonden intramolekular an die Zielsequenz und unterliegen damit einer unimolekularen Kinetik. Dagegen erfolgt beim Taqman-Verfahren die Bindung der Sonden nach einer zweimolekularen, bei dem Lightcycler-Verfahren sogar nach einer dreimolekularen Kinetik. Bei dem Lightcyler-Verfahren ist zudem eine enzymatische Degradation der Sonde erforderlich, bevor ein Signal detektiert werden kann. Schnelle PCR-Zyklen, wie sie etwa für Hoch-Durchsatz-Analysen erforderlich sind, sind daher nur eingeschränkt möglich. Dementsprechend konnte gezeigt werden, dass das Scorpion-Verfahren insbesondere unter schnellen Zyklus-Konditionen leistungsfähiger als die herkömmliche Real-Time-Methodik ist (Thelwell et al. 2000, a.a.o.). Ein weiterer Vorteil des Scorpion-Verfahrens liegt in der besonderen Spezifität. So kann durch eine Verkürzung der Sondensequenz die Spezifität derart erhöht werden, dass eine einzige Basenfehlpaarung detektierbar ist. Eine entsprechende Spezifitätserhöhung ist bei den herkömmlichen Real-Time-Varianten nicht möglich. Verkürzte Sonden führen hier vielmehr zu einer verringerten Spezifität, da die Wahrscheinlichkeit einer Bindung an unspezifische PCR-Produkte erhöht ist (vgl.: Thelwell et al. 2000, a.a.o. S. 3760).

Im folgenden ist zum ersten Mal die Anwendung des Scorpion-Verfahrens auf die Methylierungsanalyse beschrieben. Aufgrund der besonderen biologischen und medizinischen Bedeutung der Cytosinmethylierung und aufgrund der Nachteile der bekannten Verfahren stellt das Eröffnen dieser vorteilhaften, neuen Technologie einen wichtigen technischen Fortschritt dar. Neben den bereits aus der Mutationsanalyse bekannten Vorzügen des Scorpion-Verfahren ist die Anwendung der Scorpion-Methodik in der Methylierungsanalyse darüberhinaus mit weiteren Vorteilen verbunden. So ist mit den herkömmlichen PCR-Verfahren eine sensitive und spezifische Methylierungsanalyse nur bei Sequenzen möglich, in denen mehrere comethylierte Cytosin-Positionen enthalten sind. Dagegen erfordert das erfindungsgemäße Verfahren in bestimmten Ausführungsformen eine geringere Anzahl an comemethylierten Postionen. Das Scorpion-Verfahren ist in diesem Fall sequenzunabhängiger und hat daher einen breiteren Anwendungsbereich als die vergleichbaren bekannten PCR-Verfahren ("Heavy-Methyl-Verfahren", s.u.). Die Verwendung zweier Scorpion-Primer führt zu weiteren besonderen Vorteilen. So können Methylierung und Mutationen gleichzeitig untersucht werden. Zudem erlaubt die Verwendung zweier Scorpion-Primer eine interne Quantifizierung (s.u.).

### Beschreibung

Das erfindungsgemäße Verfahren erlaubt einen sensitiven Nachweis von Cytosin-Methylierungen. Es erfolgt in folgenden fünf Schritten:
1) die zu untersuchende DNA wird mit einer Chemikalie oder mit einem Enzym so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet,
2) die vorbehandelte DNA wird mittels einer Polymerase und mindestens einem Primer amplifiziert, dessen 5'-Ende über einen Linker mit einer Sonde verbunden ist,
3) das Primer-Extensionsprodukt wird von dem Matrizenstrang getrennt,
4) die Sonde hybridisiert intramolekular an das Primer-Extensionsprodukt, wobei die Hybridisierung in Abhängigkeit von dem Methylierungsstatus der DNA erfolgt,
5) es wird detektiert, ob eine Hybridisierung der Sonde stattgefunden hat.

Im ersten Schritt des erfindungsgemäßen Verfahrens wird die zu untersuchende DNA mit einer Chemikalie oder mit einem Enzym so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet. Dabei kann die zu untersuchende DNA je nach diagnostischer oder wissenschaftlicher Fragestellung aus unterschiedlichen Quellen stammen. Gemäss des vorliegenden Erfindung dienen als Ausgangsmaterial Gewebeproben, aber auch Körperflüssigkeiten, insbesondere Serum. Möglich ist auch, die DNA aus Sputum, Stuhl, Urin oder Gehirn-Rückenmarks-Flüssigkeit zu verwenden. Vorzugsweise wird die DNA zunächst aus der biologischen Probe isoliert. Die DNA-Extraktion erfolgt nach Standardmethoden, aus Blut etwa unter Verwendung des Qiagen UltraSens DNA Extraktions-Kits. Die isolierte DNA kann dann z.B. durch Umsatz mit Restriktionsenzymen fragmentiert werden. Die Reaktionsbedingungen und die in Frage kommenden Enzyme sind dem Fachmann bekannt und ergeben sich etwa aus den von den Herstellern mitgelieferten Protokollen. Anschließend wird die DNA chemisch oder enzymatisch umgewandelt. Bevorzugt erfolgt einen chemische Umsetzung mittels Bisulfit. Die Bisulfitumwandlung ist dem Fachmann in unterschiedlichen Variationen bekannt (siehe etwa: Frommer et al.: A genomic sequencing protocol that yields a positive display of 5-methylcytosine residues in individual DNA strands. Proc Natl Acad Sci U S A. 1992 Mar 1;89(5):1827-31; Olek, A modified and improved method for bisulphite based cytosine methylation analysis. Nucleic Acids Res. 1996 Dec 15;24(24):5064-6.; DE 100 29 915; DE 100 29 915). Besonders bevorzugt erfolgt die Bisulfitumwandlung in Gegenwart von denaturierenden Lösemitteln, etwa Dioxan, und eines Radikalfängers (vgl.: DE 100 29 915). In einer anderen bevorzugten Ausführungsform wird die DNA nicht chemisch, sondern enzymatisch umgewandelt. Dies ist etwa durch Einsatz von Cytidin-Deaminasen denkbar, die unmethylierte Cyidine schneller umsetzen als methylierte Cytidine. Ein entsprechendes Enzym ist kürzlich identifiziert worden (Bransteitter et al.: Activation-induced cytidine deaminase deaminates deoxycytidine on singlestranded DNA but requires the action of RNase. Proc Natl Acad Sci USA. 2003 Apr 1;100(7):4102-7).

Im zweiten Schritt des erfindungsgemäßen Verfahrens wird die vorbehandelte DNA mittels einer Polymerase und mindestens einem Primer amplifiziert. Hierzu sind dem Fachmann unterschiedliche Möglichkeiten bekannt, etwa unter Verwendung isothermer Amplifikationsverfahren. Bevorzugt sind allerdings Polymerasekettenreaktionen (PCR). Dabei sind je nach Aufbau der Primer unterschiedliche Ausführungsformen denkbar. Allen Ausführungsformen ist allerdings gemeinsam, dass bei mindestens einem der Primer das 5'-Ende über einen Linker mit einer Sonde verbunden ist. Die gesamte Sequenz aus Primer, Linker und Sonde wird - unabhängig von der Sekundärstruktur der Sonde - im folgenden als Scorpion-Primer bezeichnet. "Primer" bezeichnet dagegen nur die Primersequenz des Scorpion-Primers. Für eine methylierungsspezifische Detektion müssen entweder Sonden- oder Primersequenz methylierungsspezifisch sein. So ist es möglich, ursprünglich methylierte und unmethylierte DNA in gleicher Weise zu amplifizieren, und beide Formen erst durch Hybridisierung mit einer methylierungsspezifischen Sonde zu unterscheiden. Umgekehrt ist es auch möglich, nur eine Art der DNA zu amplifizieren und diese dann mit einer unspezifischen Sonde zu detektieren. Wird eine hohe Spezifität verlangt, so sind allerdings Verfahren bevorzugt, bei denen sowohl die Amplifikation wie auch die Detektion methylierungsspezifisch erfolgt.

Eine methylierungsspezifische Amplifikation kann auf unterschiedliche Weise erreicht werden. In einer bevorzugten Ausführungsform erfolgt die PCR unter Verwendung von Primern, die spezifisch nur an Positionen der umgewandelten Sequenz binden, die vorher entweder methyliert (oder bei umgekehrtem Ansatz: unmethyliert) waren. Dieses Verfahren ist unter dem Namen methylierungssensitive PCR bekannt (MSP). Dabei werden Primer verwendet, die mindestens ein CG-Dinukleotid enthalten; bevorzugt sind Primer, die mindestens drei CG-Positionen tragen, von denen mindestens eine am 3'-Ende lokalisiert ist. Für die Amplifikation der unmethylierten Sequenzen bzw. der Gegenstränge sind dementsprechend TG- oder CA-Dinukleotide erforderlich. Die exakten technischen Angaben zur Durchführung einer MSP sind dem Fachmann bekannt (Vgl.: Herman et al.: Methylation-specific PCR: a novel PCR assay for methylation status of CpG islands. Proc Natl Acad Sci U S A. 1996 Sep 3;93(18):9821-6; US 5,786,146; US 6,017,704; US 6,200,756).

Eine andere bevorzugte Ausführungsform zur methylierungsspezifischen Amplifikation ist die "Heavy Methyl"-Methode. Dabei wird mindestens ein methylierungsspezifisches Blocker-Oligomer eingesetzt, das an ein CG (bzw. TG oder CA)-Dinukleotid bindet und so die Amplifikation der Hintergrund-DNA verhindert. Die Ausführungsform kann über die Auswahl der Polymerase oder über die Modifikation der Blockeroligomere so ausgestaltet sein, dass ein Abbau oder eine Verlängerung der Blocker minimiert wird. Exakte technische Angaben zur Durchführung der Amplifikation sind dem Fachmann bekannt (WO 02/072880).

Die methylierungsspezifischen und die nicht-methylierungsspezifischen Amplifikationen lassen sich erfindungsgemäß mit unterschiedlichen Linker- und Sondenelementen kombinieren. Allen Ausführungsformen ist allerdings gemeinsam, dass bei mindestens einem der Primer das 5'-Ende über einen Linker mit einer Sondensequenz verbunden ist. Angaben über Länge und die Struktur des Linkers ergeben sich aus dem Stand der Technik. Insbesondere muss sichergestellt sein, dass die Sonde nicht amplifiziert wird. Dies kann über unterschiedliche Ausgestaltungen erreicht werden, die dem Fachmann bekannt sind (EP 0416817; U.S. 5,525,494; US 20030087240). In einer bevorzugten Ausführungsform enthält der Linker einen PCR-Blocker, der die Primer-Verlängerung auf dem Gegenstrang verhindert. Besonders bevorzugt sind dabei Hexaethylenglycol-(HEG)-Monomere. Alternativ kann der Linker 2-O-alkyl-RNA enthalten. Möglich ist auch die Verwendung von Oligonukleotiden, die zum Teil in 5'-5'-Richtung miteinander verbunden sind (Vgl.: US 20030087240).

Weiterhin sind die Scorpion-Primer so aufgebaut, dass die Sonde intramolekular an ein Verlängerungsprodukt des Primers binden kann. Erfindungsgemäß erfolgt die Hybridisierung der Sonde in Abhängigkeit von dem Methylierungsstatus der urspünglichen DNA. Dies bedeutet, dass nur dann eine Hybridisierung stattfindet, wenn die Matrizen-DNA an den zu untersuchenden Positionen ursprünglich methyliert oder eben unmethyliert vorlag. Eine solche methylierungsspezifische Detektion kann auf unterschiedliche Weise erfolgen. Erfolgt bereits die Amplifikation methylierungsspezifisch, so kann eine nicht-methylierungsspezifische Sonde ausreichend sein. Denn zu einer Hybridisierung der Sonde kommt es nur dann, wenn sich ein (methylierungsspezifisches) Amplifikat gebildet hat. Erfindungsgemäß bevorzugt ist allerdings die Verwendung einer methylierungsspezifischen Sonde. Diese bindet entweder nur an die ursprünglich methylierte oder unmethylierte DNA. Dementsprechend enthält die Sonde mindestens ein methylierungsspezifisches CG-, TG- oder CA-Dinukleotid. Bevorzugt sind ein bis drei entsprechende Dinukleotide. Die Sondensequenz ist bevorzugt zwischen 6 und 50 Nukleotide, besonders bevorzugt zwischen 10 und 40 Nukleotide, ganz besonders bevorzugt zwischen 15 und 30 Nukleotide lang. Dabei kann die Sonde Nukleinsäure-Analoga wie Peptid-Nukleinsäuren (PNA) oder 2'-O-alkyl-RNA enthalten. Weitere technische Angaben zur Länge und Struktur der Sonden finden sich in dem US-Patent 6,326,145 und in der US-Patentanmeldung 20030087240.

Die Sonde trägt zudem mindestens eine Markierung, mit der detektiert werden kann, ob eine Hybridisierung stattgefunden hat. Hierzu sind dem Fachmann unterschiedliche Signalsysteme bekannt. So können u.a. Fluororeszenzfarbstoff-Quencher-Paare, interkalierende Farbstoffe und Farbstoffpaare, die Signale über Fluoreszenz-Resonanz-Energie-Transfer (FRET) erzeugen, verwendet werden. Denkbar ist auch der Einsatz von Systemen, die zudem eine Anbindung der Amplifikate an eine feste Phase ermöglichen. Diese und andere Beispiele, die ebenfalls zur Methylierungsanalyse einsetzbar und daher Teil dieser Erfindung sind, sind ausführlich und mit weiteren Nachweisen in dem US-Patent 6,326,145 und in der US-Anmeldung 20030087240 (insb.: [0018]; [51] ff). beschrieben.

Die Signalsysteme bestehen bevorzugt aus zwei Komponenten. Diese sind in einer Ausführungsform so konzipiert, dass nur dann ein Signal entsteht, wenn beide Komponenten räumlich voneinander getrennt sind. Umgekehrt ist es auch möglich, dass es nur dann zu einer Signalbildung kommt, wenn sich beide Komponenten in unmittelbarer räumlicher Nähe zueinander befinden. In beiden Ausführungsformen können die Komponenten entweder durch dasselbe oder durch unterschiedliche Moleküle zur Verfügung gestellt werden. Über unterschiedliche Sekundärstrukturen der Sonde können die Komponenten in der erforderlichen räumlichen Struktur gehalten werden. Hierzu gehören etwa Haarnadeln, Schleifen, Blasen, Arme, Ellbogen und Stämme. Zum Stand der Technik gehören dementsprechend eine Vielzahl möglicher Ausführungsformen (Vgl. US 6,326,145; US 20030087240, insb. [52]ff). Diese Ausführungsformen können auch zur Methylierungsanalyse eingesetzt werden und sind daher Teil dieser Erfindung.

In einer bevorzugten Ausführungsform werden Fluoreszenzfarbstoff-Quencher-Paare verwendet. Entsprechende Paare sind dem Fachmann bekannt (siehe etwa US 20030087240 [0020] mit weiteren Nachweisen). In einer besonders bevorzugten Variante sind sowohl Quencher wie auch Farbstoff an die Sonde gebunden und befinden sich in der inaktiven Form durch eine Haarnadelbildung in unmittelbarer räumliche Nähe zueinander (vgl.: Thelwell et al 2000 a.a.o.). Exakte technische Angaben über verwendbare Haarnadelstrukturen gehören zum Stand der Technik (US 6,326,145; US 20030087240).

In einer weiteren bevorzugten Ausführungsform trägt der Scorpion-Primer zwei Signalkomponenten, die in der inaktiven Form räumlich voneinander entfernt sind, und die durch die Hybridisierung der Sonde an ein Primer-Extensionsprodukt in räumliche Nähe zueinander gebracht werden. In einer besonders bevorzugten Variante erzeugen die Signalkomponenten in der aktiven Form ein Signal über einen Fluoreszenz-Resonanz-Energie-Transfer. Dabei befindet sich die erste Komponente im 3'-Bereich und die zweite Komponente im 5'-Bereich des Scorpion-Primers.

In einer anderen besonders bevorzugten Ausführungsform sind die Signalkomponenten an unterschiedliche Moleküle gebunden und befinden sich in der inaktiven Form in räumlicher Nähe zueinander. Dies ist etwa durch Einsatz eines weiteren Oligonukleotids möglich, das mit der Sonde in der inaktiven Form eine Duplex bildet. Dabei trägt die Sonde an ihrem 5'-Ende einen Fluoreszenzfarbstoff, während das weitere Oligonukleotid an seinem 3'-Ende einen Quencher trägt. Statt eines Oligonukleotids können auch Oligomere aus Nukleinsäure-Analoga, etwa Peptid-Nukleinsäure-Oligomere verwendet werden (Vgl.: Solinas et al. a.a.o.; US 6,326,145; US 20030087240).

In weiteren besonders bevorzugten Ausführungsformen ist der Quencher ebenfalls an ein weiteres Oligonukleotid gebunden, das in der inaktiven Form eine besondere räumliche Struktur mit der Sonde bildet. Die Sonde trägt in diesem Fall zwei Farbstoffe. Sobald Sonde und weiteres Oligonukleotid voneinander getrennt werden, erfolgt zwischen den beiden Farbstoffen eine Signalbildung mittels eines Fluoreszenz-Resonanz-Energie-Transfers. Statt eines Oligonukleotids können auch Oligomere aus Nukleinsäure-Analoga, etwa Peptid-Nukleinsäure-Oligomere verwendet werden (Vgl.: Solinas et al. 2001, a.a.o. insbesondere S. 7 f und S. 6 Abb. 5).

Eine fünfte besonders bevorzugte Ausführungsform ähnelt dem Lightcycler-Verfahren und verwendet ein zusätzliches, farbstoffmarkiertes Molekül. Eine besondere räumliche Struktur zwischen weiterem Molekül und Sonde ist hier nicht erforderlich. Die Signalbildung erfolgt wiederum über einen Fluoreszenz-Resonanz-Energie-Transfer. In der aktiven Form bindet das weitere Molekül neben die Sonde an das Amplifikat, so dass die beiden Farbstoffe in unmittelbare Nähe zueinander gelangen, und so ein Signal erzeugt wird. Als weiteres Molekül wird bevorzugt ein Oligonukleotid oder ein Oligomer aus Nukleinsäure-Analoga verwendet. Die Spezifität der Nachweisreaktion kann erhöht werden, wenn die Oligonukleotid- oder Oligomersequenz methylierungsspezifisch ist, d.h. mindestens ein CG (bzw. TG oder CA)-Dinukleotid trägt. Die Sequenz ist bevorzugt 15-30 Nukleotide lang. Weitere technische Angaben ergeben sich für den Fachmann aus dem bekannten Lightcyler-Verfahren.

Alle oben beschriebenen Ausführungsformen können unter Verwendung nur eines Scorpion-Primers neben einem konventionellen Primer durchgeführt werden. Erfindungsgemäß bevorzugt ist es aber auch, wenn sowohl Forward- wie auch Reverse-Primer in der Scorpion-Form vorliegen. Ein solche Kombination kann mit vielfältigen Vorteilen verbunden sein. So ermöglicht eine erste besonders bevorzugte Ausführungsform eine Sensitivitätssteigerung der Methylierungsanalyse. Dabei erzeugen beide Scorpion-Primer in der aktiven Form die gleichen Signale. Die Sonden hybridisieren an unterschiedliche, comethylierte Positionen der DNA. Die Signale beider Scorpion-Primer addieren sich, so dass die Signalintensität verstärkt wird. Umgekehrt kann in einer zweiten besonders bevorzugten Ausführungsform die Spezifität der Nachweisreaktion erhöht werden. Dabei tragen die Scorpion-Primer unterschiedliche Signalkomponenten. Auch hier hybridisieren die Sonden an unterschiedliche comethylierte Positionen der DNA. Als positives Signal wird aber nur angesehen, wenn beide Signale gleichzeitig auftreten. Eine dritte besonders bevorzugte Ausführungsform erlaubt eine exakte interne Quantifizierung der Methylierungsanalyse. Dabei wird der eine Scorpion-Primer zur Methylierungsanalyse und der andere Scorpion-Primer zur Bestimmung der Gesamt-DNA-Menge verwendet. Dies ist etwa möglich, wenn eine nicht-methylierungsspezifische Amplifikation erfolgt und beide Scorpion-Primer unterschiedliche Signalkomponenten tragen. Die Sonde des ersten Scorpion-Primers hybridisiert dann wie oben beschrieben an methylierungsspezifische Positionen, während die Sonde des zweiten Scorpion-Primers an nicht-methylierungsspezifische Positionen bindet. Mit Hilfe des durch den zweiten Scorpion-Primer erzeugten Signals lässt sich dann die Gesamtmenge an DNA bestimmen, während das Signal des ersten Scorpion-Primers die Menge der methylierten (bzw. unmethylierten) DNA angibt. Aus beiden Signalen lässt sich exakt die Menge an methylierter DNA bestimmen. Eine vierte besonders bevorzugte Ausführungsform ermöglicht gleichzeitig eine Methylierungs- und eine Mutationsanalyse oder eine Allel-Diskriminierung. Dazu wird der erste Scorpion-Primer zur Methylierungsanalyse verwendet (s.o.) und der zweite Scorpion-Primer zur Mutationsanalyse (bzw. Allel-Diskriminierung). Dabei tragen beide Scorpion-Primer unterschiedliche Signalkomponenten. Die Sondensequenz des zweiten Scorpion-Primers ist spezifisch für eine bestimmte Mutation (bzw. ein bestimmtes Allel). Aus den erzeugten Signale lässt sich dann auf den Methylierungsstatus und auf das Vorliegen einer Mutation schließen.

Wie bereits oben erwähnt, können unterschiedliche, methylierungsspezifische oder nicht-methylierungsspezifische Amplikationsverfahren mit unterschiedlichen Linkern und verschiedenen Sondensystemen kombiniert werden. Gleichzeitig können ein oder zwei Scorpion-Primer eingesetzt werden. Hierdurch ergeben sich vielfältige Ausführungsformen, die sich zur Methylierungsanalyse eignen und daher auch als erfindungsgemäß anzusehen sind. Wie diese Verfahren durchzuführen sind, ergibt sich aus dieser Anmeldung in Verbindung mit dem Stand der Technik. Im folgenden sind bevorzugte Reaktionsbedingungen der Erfindung näher beschrieben. Wie dem Fachmann bekannt ist, können die optimalen Reaktionsbedingungen jedoch für einige Ausführungsformen variieren.

Es ist für das erfindungsgemäße Verfahren nicht erforderlich, ausschließlich Scorpion-Primer einzusetzen. Vielmehr ist es ausreichend, wenn nur ein geringer Anteil an Scorpion-Primern neben herkömmlichen Primern derselben Sequenz verwendet wird. Das optimale Verhältnis zwischen beiden Primer-Arten hängt von dem Aufbau des speziellen Assays ab und ist leicht experimentell zu bestimmen. Bei Verwendung geeigneter Signalsysteme (z.B. unterschiedlicher Fluoreszenzfarbstoffe) ist es auch möglich, mehrere Sequenzen gleichzeitig durch Einsatz unterschiedlicher Scorpion-Primer zu untersuchen (siehe etwa Thelwell et al. 2000 a.a.o.; US 20030087240). Die Scorpion-Primer können zu unterschiedlichen Zeiten im Laufe einer Amplifikationsreaktion zum Reaktionsansatz gegeben werden. So kann es ausreichend sein, wenn die Scorpion-Primer am letzten Amplifikationszyklus beteiligt sind. Dies gilt etwa für den Fall, dass eine Endpunkt-Analyse durchgeführt werden soll (vgl.: Solinas et al. 2001, a.a.o. S. 6). Vorzugsweise sind die Scorpion-Primer allerdings schon zu Beginn der Amplifkation anwesend. Dies gilt insbesondere für Real-Time-Anwendungen. Die Amplifikation erfolgt vorzugsweise über eine PCR. Dem Fachmann ist bekannt, wie Reagenzien, Konzentrationen und Temperaturzyklen zu bestimmen sind.

Im dritten Schritt des erfindungsgemäßen Verfahrens wird das Primer-Extensionsprodukt von dem Matrizenstrang getrennt. Dies ist notwendig, um eine Bindung der Sonde an das Primer-Verlängerungsprodukt zu ermöglichen. Diese Trennung erfolgt vorzugsweise durch eine Temperaturerhöhung. In einer PCR kann diese Temperaturerhöhung Teil des üblichen Temperaturzyklus sein.

Im vierten Schritt des erfindungsgemäßen Verfahrens hybridisiert die Sonde an das Primer-Extensionsprodukt, wobei die Hybridisierung in Abhängigkeit von dem Methylierungsstatus der ursprünglichen DNA erfolgt. Dies bedeutet, dass nur dann eine Hybridisierung stattfindet, wenn die Matrizen-DNA an den zu untersuchenden Positionen ursprünglich methyliert oder eben unmethyliert vorlag. Erfolgt eine nicht-methylierungsspezifische Amplifikation, so muss die methylierungsspezifische Hybrisierung durch die Sequenz der Sonde gewährleistet sein. Bei einer methylierungsspezifischen Amplifikation und Verwendung einer nicht-methylierungsspezifischen Sonde erfolgt nur dann eine Bindung, wenn sich überhaupt ein (methylierungsspezifisches) Produkt gebildet hat (s.o.). Erfolgt die Reaktion mittels zweier Scorpion-Primer, so kann die Hybridisierung der Sonde des zweiten Scorpion-Primers unabhängig vom Methylierungsstatus erfolgen, etwa wenn die Gesamt-DNA-Menge bestimmt und neben der Methylierungauch eine Mutationsanalyse durchgeführt werden soll (s.o.).

Im fünften Schritt des erfindungsgemäßen Verfahrens wird detektiert, ob eine Hybridisierung stattgefunden hat. Dabei erfolgt die Detektion in Abhängigkeit von der Signalbildung nach dem Stand der Technik.

Dem Fachmann ist bekannt, dass die Schritte 2 bis 5 des erfindungsgemäßen Verfahrens mehrmals wiederholt werden können. Dies gilt insbesondere für Real-Time-Anwendungen.

Wie bereits mehrfach erwähnt, ist das erfindungsgemäße Verfahren in vielfältigen Ausführungsformen anwendbar. Im folgenden sind einige bevorzugte Ausführungsformen näher beschrieben. Die exakten technischen Angaben zur Ausführung dieser Verfahren erschließen sich dem Fachmann aus dem Stand der Technik in Kombination mit dieser Anmeldung. Gleichwohl ist das erfindungsgemäße Verfahren nicht auf die unten dargestellten Varianten beschränkt. Vielmehr sind auch die anderswo offenbarten Ausführungsformen der Scorpion-Technologie für die Methylierungsanalyse anwendbar und damit Teil dieser Erfindung (Vgl. insbesondere: Thelwell et al a.a.o; Solinas et al a.a.o; US 6,326,145; US 20030087240, insb. [52]ff).

Eine erste bevorzugte Ausführungsform wird im folgenden als "Methyl-Hairpin" bezeichnet. Dabei erfolgt die Amplifikation der chemisch oder enzymatisch vorbehandelten DNA mittels einer nicht-methylierungsspezifischen PCR. Dementsprechend werden Primer eingesetzt, die an ursprünglich methylierte und an ursprünglich unmethylierte DNA in gleicher Weise binden. Die Primer enthalten daher keine CG-Dinukleotide und nur solche TG- und CA-Dinukleotide, die Positionen entsprechen, die bereits vor der Umwandlung in dieser Sequenz vorlagen. Bevorzugt werden als Primerpaar ein Scorpion-Primer und ein herkömmlicher PCR-Primer eingesetzt. Der Linker des Scorpion-Primer enthält bevorzugt Hexaethylenglycol (HEG) Monomere, um eine Amplifikation der Sonde zu verhindern. Dem Fachmann sind weitere Möglichkeiten bekannt, wie das Linkerelement ausgestaltet werden kann (EP 0416817; U.S. 5,525,494; US 20030087240). Das Sondenelement des Scorpion-Primers bildet eine Haarnadelstruktur. Die Sondensequenz ist bevorzugt zwischen 6 und 50 Nukleotide, besonders bevorzugt zwischen 10 und 40 Nukleotide, ganz besonders bevorzugt zwischen 15 und 30 Nukleotide lang. Dabei kann die Sonde Nukleinsäure-Analoga wie Peptid-Nukleinsäuren (PNA) oder 2'-O-alkyl-RNA enthalten. Exakte technische Angaben über Länge und Struktur verwendbarer Haarnadelstrukturen gehören zum Stand der Technik (US 6,326,145; US 20030087240). Die Sondensequenz ist methylierungsspezifisch, d.h. sie trägt mindestens ein CG-Dinukleotid bzw. methylierungsspezifische TG- und CA-Dinukleotide. Bevorzugt sind ein bis drei entsprechende Dinukleotide. Die Sonde trägt zudem ein Fluoreszenzfarbstoff-Quencher-Paar, das sich durch die Haarnadelbildung in unmittelbarer räumlicher Nähe befindet. Im Zuge der PCR erfolgt eine Verlängerung des Scorpion-Primers und anschließend eine thermische Trennung des verlängerten Primers von dem Matrizenstrang. Die Sonde des Scorpion-Primers bindet dann in Abhängigkeit von dem Methylierungsstatus intramolekular an den verlängerten Primer. Durch die Trennung von Farbstoff und Quencher entsteht ein Fluoreszenz-Signal. Die Detektion des Signals kann nach dem Stand der Technik auf unterschiedliche Weise erfolgen. Es ist für das erfindungsgemäße Verfahren ausreichend, nur einen geringen Anteil an Scorpion-Primern neben herkömmlichen Primern derselben Sequenz einzusetzen (s.o.). Bei Verwendung geeigneter Signalsysteme ist es möglich, mehrere Sequenzen gleichzeitig durch Einsatz unterschiedlicher Scorpion-Primer zu untersuchen. In Beispiel 1 ist ein Methyl-Hairpin-Verfahren beschrieben. Beispiel 2 zeigt einen Vergleich zwischen der MethyLight und der Methyl-Hairpin-Technologie. In Figur 5 ist der Ablauf einer Methyl-Hairpin-Reaktion grob schematisch dargestellt.

Eine zweite bevorzugte Ausführungsform wird im folgenden als "MSP-Methyl-Hairpin" bezeichnet. Dabei erfolgt die Amplifikation der chemisch oder enzymatisch vorbehandelten DNA mittels einer methylierungsspezifischen PCR. Dementsprechend werden Primer eingesetzt, die bevorzugt entweder an die ursprünglich methylierte oder an die ursprünglich unmethylierte DNA binden. Die Primer enthalten daher CG-Dinukleotide oder methylierungsspezifsche TG- oder CA-Dinukleotide. Bevorzugt tragen die Primer mindestens drei entsprechende Dinukleotide, von denen mindestens eines am 3'-Ende lokalisiert ist. Bevorzugt werden als Primerpaar ein Scorpion-Primer und ein herkömmlicher PCR-Primer eingesetzt. Linker und der Sonde sind so aufgebaut wie im Methyl-Hairpin-Verfahren (s.o.). Dabei verfügt die Sonde bevorzugt über eine methylierungsspezifische Sequenz, d.h. sie trägt mindestens ein CG-Dinukleotid bzw. methylierungsspezifische TG- oder CA-Dinukleotide. Bevorzugt sind ein bis drei entsprechende Dinukleotide. Da die Amplifikation bereits methylierungsspezifisch erfolgt, ist es auch möglich, eine nicht-methylierungsspezifische Sonde einzusetzen. Die Signalerzeugung erfolgt wie bei dem Methyl-Hairpin-Verfahren über ein Fluoreszenzfarbstoff-Quencher-Paar (s.o.).

Eine dritte bevorzugte Ausführungsform wird im folgenden als "Heavy Methyl-Hairpin" bezeichnet. Dabei erfolgt die Amplifikation der chemisch oder enzymatisch vorbehandelten DNA mittels einer methylierungsspezifischen Heavy Methyl-PCR (s.o.). Hierzu werden nicht-methylierungsspezifische Primer eingesetzt, d.h. Primer, die an ursprünglich methylierte und an ursprünglich unmethylierte DNA in gleicher Weise binden. Die Primer enthalten daher keine CG-Dinukleotide und nur solche TG- und CA-Dinukleotide, die Positionen entsprechen, die bereits vor der Umwandlung in dieser Sequenz vorlagen. Die methylierungsspezifische Amplifikation wird durch Einsatz mindestens eines methylierungsspezifisches Blocker-Oligomers sichergestellt. Der Blocker bindet an ein CG- (bzw. an ein methylierungsspezifisches TG- oder CA-) Dinukleotid und verhindert so die Amplifikation der Hintergrund-DNA. Die Ausführungsform kann über die Auswahl der Polymerase oder über die Modifikation der Blockeroligomere so ausgestaltet sein, dass ein Abbau oder eine Verlängerung der Blocker minimiert wird. Bevorzugt werden als Primerpaar ein Scorpion-Primer und ein herkömmlicher PCR-Primer eingesetzt. Linker und Sonde sind so aufgebaut wie im Methyl-Hairpin-Verfahren (s.o.). Dabei verfügt die Sonde bevorzugt über eine methylierungsspezifische Sequenz, d.h. sie trägt mindestens ein CG-Dinukleotid bzw. methylierungsspezifische TG- oder CA-Dinukleotide. Bevorzugt sind ein bis drei entsprechende Dinukleotide. In einer besonders bevorzugten Ausführungsform verwendet der Heavy Methyl-Hairpin-Assay für das Blocken und für die Signalerzeugung dieselben methylierungsspezifischen Dinukleotide. Hierdurch wird die Anzahl der zur Analyse erforderlichen Dinukleotide verringert, so dass das HeavyMethyl-Hairpin-Verfahren eine breitere Anwendbarkeit als das Heavy Methyl-Verfahren haben kann. Zudem wird auch eine erhöhte Spezifität erreicht. Da die Amplifikation bereits methylierungsspezifisch erfolgt, ist es möglich, eine nicht-methylierungsspezifische Sonde nicht-methylierungsspezifische Sonde einzusetzen. Die Signalerzeugung erfolgt wie bei dem Methyl-Hairpin-Verfahren über ein Fluoreszenzfarbstoff-Quencher-Paar (s.o.). In Beispiel 3 ist ein Heavy Methyl-Hairpin-Verfahren beschrieben.

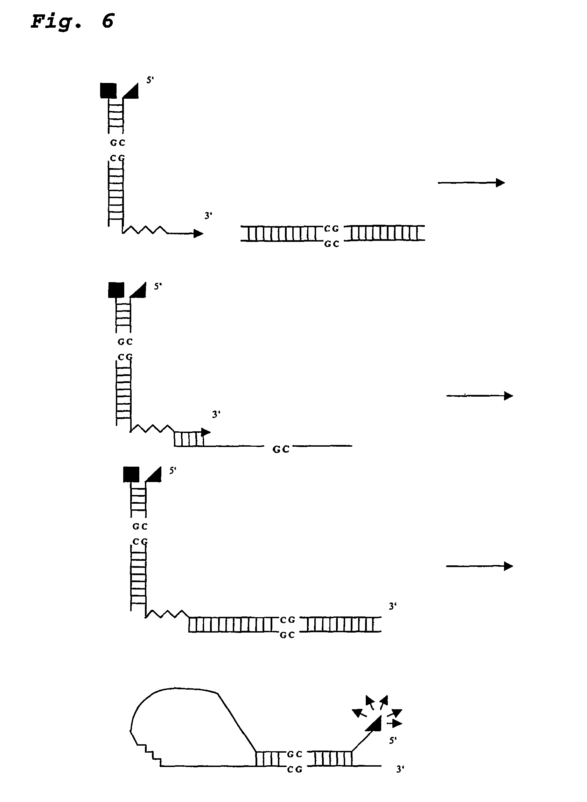

Eine vierte bevorzugten Ausführungsform wird im folgenden als "Methyl-Duplex" bezeichnet. Diese Variante unterscheidet sich von der oben beschriebenen Methyl-Hairpin-Variante nur durch das Sondenelement. Im Methyl-Duplex-Verfahren sind Farbstoff und Quencher an unterschiedliche Moleküle gebunden und befinden sich in der inaktiven Form in räumlicher Nähe zueinander. Dies ist etwa durch Einsatz eines weiteren Oligonukleotids möglich, das mit der Sonde in der inaktiven Form eine Duplex bildet. Statt eines Oligonukleotids können auch andere Oligomere aus Nukleinsäure-Analoga, etwa Peptid-Nukleinsäure-Oligomere verwendet werden. (Vgl.: Solinas et al. a.a.o.; US 6,326,145; US 20030087240). In Figur 6 ist der Ablauf einer Methyl-Hairpin-Reaktion grob schematisch dargestellt.

Eine fünfte bevorzugte Ausführungsform wird im folgenden als "MSP-Methyl-Duplex" bezeichnet. Diese Variante unterscheidet sich von der oben beschriebenen MSP-Methyl-Hairpin-Variante nur durch das Sondenelement. Dieses bildet wiederum mit einem weiteren Oligomer eine besondere räumliche Struktur, etwa eine Duplex (s.o.).

Eine sechste bevorzugte Ausführungsform wird im folgenden als "Heavy Methyl-Duplex" bezeichnet. Diese Variante entspricht einer Kombination aus dem "Heavy Methyl-Hairpin" mit einem Sondenelement, das mit einem weiteren Oligomer eine besondere räumliche Struktur, etwa eine Duplex bildet (s.o).

Bei einer siebten besonders bevorzugten Ausführungsform handelt es sich um einen quantitativen "Methyl-Hairpin" Assay. Diese Ausführungsform entspricht dem "Methyl-Hairpin-Assay", wobei ein zweiter Scorpion-Primer eingesetzt wird. Hierdurch ist eine interne Quantifizierung möglich. Die Sonde des zweiten Scorpion-Primers ist nicht-methylierungs-spezifisch und bindet unabhängig vom Methylierungsstatus an die Amplifikate. Mit Hilfe des hierdurch erzeugten Signals lässt sich dann die Gesamtmenge an DNA bestimmen, während das Signal des ersten Scorpion-Primers die Menge der methylierten (bzw. unmethylierten) DNA ergibt. Aus beiden Signalen lässt sich exakt die Menge an methylierter DNA bestimmen.

Ein weiterer Aspekt der Erfindung besteht in der Verwendung aller erfindungsgemäßen Ausführungsformen. Werden krankheitsspezifische Cytosinpositionen untersucht, so eignet sich das erfindungsgemäße Verfahren insbesondere zur Diagnose oder Prognose von Krebserkrankungen oder anderen mit einer Veränderung des Methylierungsstatus assoziierten Krankheiten. Hierzu gehören u.a. CNS-Fehlfunktionen, Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion. Das erfindungsgemäße Verfahren eignet sich außerdem zur Vorhersage von unerwünschten Arzneimittelwirkungen, zur Festlegung einer spezifischen Arzneimitteltherapie (personalisierte Medizin) und zur Überwachung des Erfolges einer Arzneimitteltherapie. Eine weitere Anwendung ist die Unterscheidung von Zelltypen oder Geweben und die Untersuchung der Zelldifferenzierung.

Gegenstand der Erfindung ist auch die Verwendung von Scorpion-Primern zur Methylierungsanalyse und zum Nachweis oder zur Prognose der oben genannten, mit einer Veränderung des Methylierungsstatus assoziierten Krankheiten. Ein weiterer Gegenstand der Erfindung ist die Verwendung der Scorpion-Primer zur Vorhersage von unerwünschten Arzneimittelwirkungen, zur Festlegung einer spezifischen Arzneimitteltherapie (personalisierte Medizin) und zur Überwachung des Erfolges einer Arzneimitteltherapie. Ferner ist Gegenstand der Erfindung ist die Verwendung der Scorpion-Primer zur Unterscheidung von Zelltypen oder Geweben und zur Untersuchung der Zelldifferenzierung. Unter Scorpion-Primer wird dabei ein Primer verstanden, der an seinem 5'-Ende über einen Linker mit einer Sondensequenz verbunden ist (s.o.).

Schließlich ist Gegenstand der vorliegenden Erfindung ist auch ein Kit, bestehend aus mindestens einem Scorpion-Primer, einer Polymerase und den erforderlichen Reagenzien für eine PCR.

### Beispiele

### Beispiel 1: Quantifizierung methylierter DNA des brcal-Gens mit einem Methyl-Hairpin-Real-Time-PCR-Assay.

Es wurde ein Bisulfit-behandeltes Fragment des menschlichen brca1-Gens amplifiziert (Genbank Accession: L78833.1, nt 3538 - nt 3666; SeqID-7). Als Matrize wurde menschliche DNA aus peripheren Blut-Zellen (Roche Diagnostics) und umfassend methylierte menschliche DNA (Serologicals) eingesetzt. Beide DNA-Arten wurden mit einer Bisulfit-Lösung behandelt (Olek et al. 1996, a.a.o.). Hierdurch wurden unmethylierte Cytosine in Uracil umgewandelt, während 5-Methylcytosine unverändert blieben. Nach der Bisulfit-Behandlung wurde die DNA-Konzentration mittels UV-Absorption (260 nm) bestimmt. Die Leistungsfähigkeit des Assays wurde durch Einsatz von 10 ng, 1 ng und 0,1 ng Bisulfit-behandelter methylierter Matrizen-DNA untersucht. Die Reaktion wurde in einem Gesamtvolumen von 20 µl unter Verwendung eines Light-Cycler-Gerätes durchgeführt. Der Reaktionsansatz enthielt 10 µl Matrizen-DNA (Konzentrationsangaben siehe unten), 2 µl des FastStart-LightCycler-Reaktionsgemisches für Hybridisierungssonden (Roche Diagnostics), 0,30 µmol/l Forward Primer (5'-GAAGtTGAtAGATGGGTATTtTTTGA-3'; SeqID-1), 0,10 µmol/l Reverse Primer (5'-CCCCCTTCCTaATCCTCAa-3'; SeqID-2), 0,5 µmol/l Scorpion-Primer (5'-FAM-GGCAGCCTAGGTCGCGAGGGAAGGCTGCC-MR-HEG-CCCCCTTCCTaATCCTCAa-3'; Sondenelement: SeqID-6; Primerelement: SeqID-8) und 2mmol/l MgCl₂. Die Thermozyklen begannen mit einer Inkubation bei 95°C für 10 Minuten, gefolgt von 55 Zyklen mit folgenden Schritten: 95° C für 10 Sekunden, 56°C für 30 Sekunden und 72°C für 10 Sekunden. Die Fluoreszenz wurde in jedem Zyklus vor der Annealing-Phase bei 56 °C detektiert.

Wegen der identischen Basenpaarungseigenschaften von Uracil und Thymin wurden die Positionen, die den umgewandelten, nicht methylierten Cytosinen entsprechen, mit einem kleinen "t" gekennzeichnet (bzw. kleines "a" im komplementären Strang). Dagegen stehen das große "T" (bzw. "A" im komplementären Strang) für die bereits vor der Bisulfit-Behandlung vorhandenen Thymine. Die Abkürzungen in den Primersequenzen stehen hier und in den weiteren Beispielen für folgendes: FAM = Fluoresceine-Label; red640 = LightCycler-Fluorescence-Label für Kanal F2; MR = Methylred-Label; HEG = Hexaethylenglycol-Spacer; pho=Phosphat-Modifikation; fluo= Fluoresceine.

Die Ergebnisse sind in Tabelle 1 und in Figur 1 gezeigt. Tabelle 1 zeigt den von der Lightcycler-Software berechneten Mittelwert der Schwellenwerte von zwei Wiederholungen. Die Negativkontrollen ergaben keine Amplifikate (10 ng unmethylierte Bisulfit-behandelte DNA; 50 ng genomische DNA; Wasser). Die Daten zeigen, dass mit dem Methyl-Hairpin-Assay ein spezifischer Nachweis der methylierten, Bisulfit-behandelten DNA des brcal-Gens möglich ist. Bei der Bisulfit-behandelten methylierten Matrizen-DNA zeigt die PCR eine Linearität von mindestens drei Größenordnungen (Figur 1). Der Methyl-Hairpin-Assay ist daher in der Lage, methylierte DNA auf Real-Time-PCR-Plattformen zu quantifizieren.

**Tabelle 1: Leistungsfähigkeit des brcal-Methyl-Hairpin-Assays zum Nachweis methylierter DNA. Die Schwellenzyklen wurden mit der LightCycler-Software aus zwei Wiederholungen erhalten (Methode: second derivtate maximum).**

| Menge an Bisulfit-behandelter Matrizen DNA (ng) | Schwellenzyklen bei Methyl-Hairpin |
|---|---|
| 10 | 34,9 |
| 1 | 37,9 |
| 0,1 | 40,8 |
| 0,0 | 0 |

### Beispiel 2: Vergleich eines Methyl-Hairpin-Assays mit einem MethyLight-Assay zur methylierungsspezifischen Detektion des brcal-Amplicons in DNA-Mischungen aus methylierter und unmethylierter, Bisulfit-behandelter DNA.

Im folgenden wurden die Real-Time-PCR-Verfahren Methyl-Hairpin und MethyLight verglichen. Bestimmt wurde die Menge an ursprünglich methylierter, Bisulfit-behandelter DNA vor einem Hintergund ursprünglich unmethylierter, Bisulfit-behandelter DNA.
Die PCR-Amplifikationen wurden in einem Gesamtvolumen von 20 µl unter Verwendung eines LightCycler-Gerätes (Roche Diagnostics) durchgeführt. Amplifiziert wurde ein Bisulfit-behandeltes DNA-Fragment des menschlichen brca1-Gens (nt 3538 - nt 3666 in Genbank Accession No. L78833.1; SeqID-7) Der Methyl-Hairpin-Reaktionsmix enthielt 10 µl Matrizen-DNA (Konzentrationen siehe unten), 2 µl des FastStart-LightCycler-Reaktion-Mixes für Hybridisierungssonden (Roche Diagnostics), 0,30 µmol/l Forward Primer (5'-GAAGtTGAtAGATGGGTATTtTTTGA-3'; SeqID-1), 0,10 µmol/l Reverse Primer (5'-CCCCCTTCCTa.ATCCTCAa-3'; SeqID-2), 0,5 µmol/l Scorpion-Primer (5'-FAM-GGCAGCCTAGGTCGCGAGGGAAGGCTGCC-MR-HEG-CCCCCTTCCTaATCCTCAa-3'; Sondenelement: SeqID-6; Primerelement: SeqID-8) und 2 mmol/l MgCl₂. Der MethyLight-Reaktionsmix enthielt 10 µl Matrizen-DNA, 2 µl des FastStart-LightCycler-Reaktionsmixes für Hybridizierungssonden (Roche Diagnostics), 0,30 µmol/l Forward Primer (5'-GAAGtTGAtAGATGGGTATTtTTTGA-3'; SeqID-1), 0,30 µmol/l Reverse Primer (5'-CCCCCTTCCTaATCCTCAa-3'; SeqID-2), 0,25 µmol/l Hybridisierungssonde 1 (5'-GCGGAAttTGAGAGGCGTA-fluo-3'; SeqID-3) und Hybridisierungssonde 2 (5'-red640-GCGTTGTGAAtttTGGGGAG-pho-3'; SeqID-4). Die Thermozyklen begannen mit einer Inkubation bei 95°C für 10 Minuten, gefolgt von 55 Zyklen mit den folgenden Schritten: 95°C für 10 Sekunden, 56°C für 30 Sekunden und 72°C für 10 Sekunden. Die Fluoreszenz-Signale wurden in jedem Zyklus vor der Annealing-Phase bei 56°C detektiert.

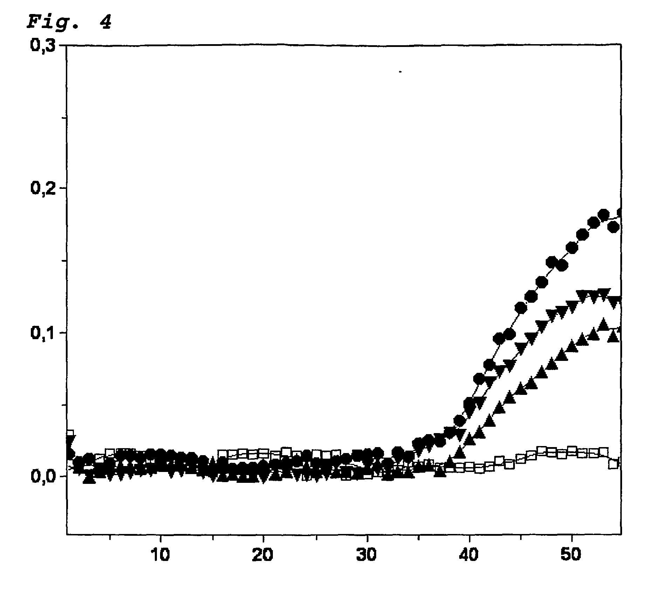

Die DNA wurde aus derselben Quelle wie in Beispiel 1 beschrieben isoliert. In jeder Reaktion wurde die Gesamtmenge von 20 ng Bisulfit-behandelter DNA eingesetzt. Die Anteile methylierter DNA betrugen 100 %, 50 % und 10 %. Das entspricht in absoluten Mengen 20 ng (100%), 10 ng (50%) und 2 ng (10%) pro Reaktion. Beide Assays wurden parallel in demselben LightCycler-Gerät mit denselben DNA-Mischungen durchgeführt. Die Fluoreszenz-Signale wurden in unterschiedlichen Kanälen detektiert. Der Methyl-Hairpin-Assay generierte Signale im Kanal F1 (520 nm). Die Signale des MethyLight-Assays wurden in Kanal F2 nachgewiesen (640nm).
Die experimentellen Daten zeigen, dass bei allen Konzentrationen mit beiden Assays Signale methylierter DNA vor einem Hintergrund unmethylierter DNA erhalten werden können (Figuren 2 und 3). Unmethylierte Bisulfit-behandelte DNA ergab keine Signale. Der Methyl-Hairpin-Assay generierte im Vergleich zum MethyLight-Assay höhere Signale und erlaubte eine bessere Analyse. Dies gilt insbesondere für die 10%-Proben. Das Signal des Methyl-Light-Assays liegt dabei zu 140%, das Signal des Scorpion-Assays zu 180% über dem Hintergrundsignal. Die Ergebnisse zeigen, dass der Methyl-Hairpin-Assay in der Lage ist, zumindest 2 ng methylierter DNA bei einem Hintergrund von 18 ng unmethylierter DNA nachzuweisen. Dabei besaß er eine höhere Sensitivität als der MethyLight-Assay.

### Beispiel 3: Verwendung des Heavy Methyl-Hairpin-Assay zum Nachweis methylierter DNA des brcal-Gens bei 4000-fach höherem Hintergrund an unmethylierter DNA.

Der hier beschriebene Assay verbindet die Vorteile der Heavy Methyl- und der Methyl-Hairpin Technologie. Die Verwendung methylierungsspezifischer Blocker (Heavy Methyl) und methylierungsspezifischer Detektion mit Scorpion-Primern (Methyl-Hairpin) schafft eine neue Real-Time-PCR-Technologie für eine sensitive Detektion methylierter DNA (Heavy Methyl-Hairpin).

Der Heavy Methyl-Hairpin-Assay wurde in einem Gesamtvolumen von 20 µl unter Verwendung eines LightCycler-Gerätes (Roche Diagnostics) durchgeführt. Es wurde ein Bisulfit-behandeltes DNA-Fragment des humanen brca1-Gens amplifiziert (nt 3538 - nt 3666 in Genbank Accession No. L78833.1; SeqID-7). Das Reaktionsgemisch enthielt 10 µl Matrizen-DNA (Konzentrationen siehe unten), 2 µl FastStart-LightCycler-Reaktionsmix für Hybridisoierungssonden (Roche Diagnostics), 0,30 µmol/l Forward Primer (5'-GAAGtTGAtAGATGGGTATTtTTTGA-3'; SeqID-1), 0,10 µmol/l Reverse Primer (5'-CCCCCTTCCTaATCCTCAa-3'; SeqID-2), 0,5 µmol/l Scorpion-Primer (5'-FAM-GGCAGCCTAGGTCGCGAGGGAAGGCTGCC-MR-HEG-CCCCCTTCCTaATCCTCAa-3'; Sondenelement: SeqID-6; Primerelement: SeqID-8), 4 µmol/l Blocker (5'-TAATCCTCAaCACTTCCCTCACAACCT-pho-3'; SeqID-5) und 2mmol/l MgCl₂.
Die Thermozyklen begannen mit einer Inkubation bei 95°C für 10 Minuten, gefolgt von 55 Zyklen mit folgenden Schritten: 95°C für 10 Sekunden, 56°C für 30 Sekunden und 72°C für 10 Sekunden. Die Fluoreszenz-Signale wurden in jedem Zyklus vor der Annealing-Phase bei 56°C detektiert. Die DNA-Gemische wurden aus derselben Quelle isoliert wie in Beispiel 1 beschrieben. Die Menge an ursprünglich methylierter, Bisulfit-behandelter DNA betrug in jeder Probe 100 pg. Die Menge an unmethylierter Hintergrund-DNA variierte jeweils. Verhältnisse methylierter zu unmethylierter DNA von 1:1000 und 1:4000 wurden durch entsprechende Mischungen nach der Bisulfit-Behandlung hergestellt. Als absolute DNA-Mengen ergaben sich für die Reaktionen dementsprechend 100,1ng (1:1000) bzw. 400,1ng (1:4000). Als positive Kontrolle wurden 100 pg methylierte DNA ohne unmethylierten Hintergrund und als negative Kontrolle 100 ng unmethylierte DNA verwendet.

Die Ergebnisse sind in Figur 4 gezeigt. 100 pg methylierter DNA konnten mit dem Heavy Methyl-Hairpin-Assay in den beiden relativen Sensitivitäten von 1:1000 und 1:4000 nachgewiesen werden. Keine Signale wurden bei 100 ng unmethylierter DNA erhalten. Dagegen ergab die Positivkontrolle mit 100 pg methylierter DNA ohne Hintergrund unmethylierter DNA das stärkste Signal. Die Daten zeigen, dass der Heavy Methyl-Hairpin-Assay eine sensitive Detektion methylierter DNA vor einem 4000-fachen Hintergrund unmethylierter DNA gewährleisten kann.

### Beispiel 4 (theoretisches Beispiel): Methylierungsanalyse des GSTPi Gens durch Scorpio Real Time Analyse (Heavy Methyl-Hairpin-Assay)

Folgendes (bisulfit behandeltes) Fragment des GSTPi Gens wird amplifiziert: CGGGAttAtttTTATAAGGtTCGGAGGtCGCGAGGttTTCGtTG-GAGTTTCGtCGtCGtAGTtTTCGttAttAG (SEQ ID NO:9 nt; 1845 - nt 1924 in in Genbank Accession No. AY324387). Die PCR wird mit den in den oben beschriebenen Reaktionsbedingungen durchgeführt. Als Primer werden folgende Sequenzen verwendet: Forward primer (GGGAttAtttTTATAAGGtT; SEQ ID NO:10), Reverse primer (TACTCACTaaTaaCKAAaACTaC; SEQ ID NO:11), Scorpion primer (FAM- ggcagccGtTG-GAGtttCGtCGggctgcc-DDQ-HEG-TACTCACTAATAACKAAAACTAC; SEQ ID NO:12, 13), 4µM Blocking probe (CTAATAACaAAAACTACaA-CaACaAAACTCCAAC-PHO; SEQ ID NO:14). Statt des oben beschriebenen Standard Scorpio Primer kann auch folgender Duplex Scorpio Primer verwendet werden: FAM-GtTGGAGtttCGtCG-HEG-TACTCACTAATAACKAAAACTAC (Seq ID; CGaCGaaaCTCCAaC-DDQ; Seq ID NO:15; NO:16).

### Erläuterungen zu den Figuren

Figur 1: Real-Time-Amplifizierung der Bisulfit-behandelten brca1-DNA mittels des Methyl-Hairpin-Assays (Bsp.1). Die Y-Achse zeigt das in jedem Zyklus gemessene Fluoreszenz-Signal (Kanal F1). Die X-Achse zeigt die Anzahl der Zyklen. Kein Signal ergaben die NegativKontrollen: Wasser (Stern), 50 ng genomische DNA (diagonales Kreuz); 10 ng unmethylierte, Bisulfit-behandelte DNA (leerer Kreis). Die methylierte, Bisulfit-behandelte DNA ist mit schwarzen Kreisen (10 ng), schwarzen Dreiecken (1 ng) und schwarzen Rechtecken (0,1 ng) gekennzeichnet.
Figur 2: Real-Time-Amplifizierung der Bisulfit-behandelten brcal-DNA mit dem MethylLight-Assay unter Verwendung von Hybridisierungssonden (Bsp. 2). Die Y-Achse zeigt das Fluoreszenz-Signal in Kanal F2 (640nm). Die Messung erfolgte in jedem Zyklus (X-Achse). Die Kurven zeigen 100% (schwarze Kreise), 50% (schwarze Rauten) und 10% methylierte DNA (schwarze Dreiecke) vor einem Hintergrund unmethylierter DNA. Insgesamt wurden 20 ng Bisulfit-behandelter DNA in jedem Ansatz untersucht. Bei 20 ng unmethylierter DNA konnte kein Signal bestimmt werden (leere Rechtecke).
Figur 3: Real-Time-Amplifizierung der Bisulfit-behandelten brcal-DNA mit dem MethylLoop-Assay unter Verwendung von Hybridisierungssonden (Bsp.2). Die Y-Achse zeigt das Fluoreszenz-Signal in Kanal F1 (520 nm). Die Messung erfolgte in jedem Zyklus (X-Achse). Die Kurven zeigen 100% (schwarze Kreise), 50% (schwarze Rauten) und 10% methylierter DNA (schwarze Dreiecke) vor einem Hintergrund unmethylierter DNA. Insgesamt wurden pro Ansatz 20 ng Bisulfit-behandelter DNA verwendet. Bei 20 ng unmethylierter DNA wurde kein Signal detektiert (leere Rechtecke).
Figur 4: Sensitive Detektion der brca1-DNA mit dem Heavy Methyl-Hairpin-Assay (Bsp.3). Die Y-Achse zeigt das Fluoreszenz-Signal in Kanal F1 (520nm). Die Messung erfolgte in jedem Zyklus (X-Achse). Bestimmt wurden jeweils 100 pg methylierter DNA mit folgenden Mengen an unmethylierter Hintergrund-DNA: ohne Hintergrund-DNA (schwarze Kreise), 100 ng (schwarze Dreiecke mit Spitze nach unten), 400 ng /schwarze Dreiecke mit Spitze nach oben). Bei der Negativkontrolle (100 ng unmethylierter DNA ohne methylierter DNA) wurde kein Signal erhalten (leere Rechtecke).
Figur 5 zeigt grob schematisch den Ablauf des Methyl-Hairpin-Verfahrens. Der Scorpion-Primer befindet sich auf der linken Seite. Das (methylierungsspezifische) Sondenelement des Scorpion-Primer liegt in Form einer Haarnadel vor. Es trägt einen Fluoreszenz-Farbstoff (schwarzes Dreieck) und einen Quencher (schwarzes Rechteck), die sich durch die Haarnadelstruktur in unmittelbarer räumlicher Nähe zueinander befinden. Die Zick-Zack-Linie kennzeichnet den PCR-Stopper innerhalb des Linkerelements. Die zu untersuchende, bereits bisulfitierte DNA ist in Form eines Doppelbalkens dargestellt. Untersucht werden soll eine CG-Position innerhalb der DNA. Nur wenn das Cytosin ursprünglich methyliert war, hat es die Bisulfitbehandlung unverändert überstanden. Unmethylierte Cytosine werden in Uracil (bzw. Thymin) konvertiert. Nach einer thermischen Denaturierung kann das (hier nicht-methylierungsspezifische) Primerelement des Scorpion-Primers an die einzelsträngige DNA binden. Mit Hilfe einer Polymerase kommt es zu einer Primerverlängerung. Nach einer weiteren thermischen Denaturierung bindet die Sonde intramolekular an den verlängerten Primer, sofern das Verlängerungsprodukt ein CG-Dinukleotid enthält. Dies ist aber nur dann der Fall, wenn die zu untersuchende DNA ursprünglich methyliert vorlag. Unmethylierte Cytosine führen zu TG-Dinukleotide, an die keine Sondenbindung erfolgt (nicht gezeigt). Durch die Bindung der Sonde an die verlängerte Primer-Sequenz wird die Haarnadel aufgelöst und ein Fluoreszenz-Signal kann detektiert werden werden (Figur modifiziert nach Thelwell et al 2000, a.a.o., S. 3753).
Figur 6 zeigt grob schematisch den Ablauf des Methyl-Duplex-Verfahrens. Der Scorpion-Primer befindet sich auf der linken Seite. Das (methylierungsspezifische) Sondenelement des Scorpion-Primer liegt zusammen mit einem weiteren Oligonukleotid in Form eines Doppelstrangs vor. Das Sondenelement trägt einen Fluoreszenz-Farbstoff (schwarzes Dreieck). An das weitere Oligonukleotid ist ein Quencher gebunden (schwarzes Rechteck). Beide Komponente befinden sich durch die Duplex in unmittelbarer räumlicher Nähe zueinander befinden. Die Zick-Zack-Linie kennzeichnet den PCR-Stopper innerhalb des Linkerelements des Scorpion-Primers. Die zu untersuchende, bereits bisulfitierte DNA ist in Form eines Doppelbalkens dargestellt. Untersucht werden soll eine CG-Position innerhalb der DNA. Nur wenn das Cytosin ursprünglich methyliert war, hat es die Bisulfitbehandlung unverändert überstanden. Unmethylierte Cytosine werden in Uracil (bzw. Thymin) konvertiert. Nach einer thermischen Denaturierung kann das (hier nicht-methylierungsspezifische) Primerelement des Scorpion-Primers an die einzelsträngige DNA binden. Mit Hilfe einer Polymerase kommt es zu einer Primerverlängerung. Nach einer weiteren thermischen Denaturierung wird die Duplex aus Sonde und weiterem Oligonukleotid getrennt und die Sonde bindet intramolekular an den verlängerten Primer, sofern das Verlängerungsprodukt ein CG-Dinukleotid enthält. Dies ist aber nur dann der Fall, wenn die zu untersuchende DNA ursprünglich methyliert vorlag. Unmethylierte Cytosine führen zu TG-Dinukleotiden, an die keine Sondenbindung erfolgt (nicht gezeigt). Durch die Bindung der Sonde an die verlängerte Primer-Sequenz werden Farbstoff und Quencher getrennt, und ein Fluoreszenz-Signal kann detektiert werden (Figur modifiziert nach Solinas et al 2001, a.a.o., S. e96).

## Patentansprüche

1. Verfahren zum Nachweis von Cytosin-Methylierungen in DNA aus Gewebeproben oder Körperflüssigkeiten, **dadurch gekennzeichnet, dass**
a) die zu untersuchende DNA mit einer Chemikalie oder mit einem Enzym so umgesetzt wird, dass 5-Methylcytosin unverändert bleibt, während unmethyliertes Cytosin in Uracil oder in eine andere Base umgewandelt wird, die sich im Basenpaarungsverhalten von Cytosin unterscheidet,
b) die vorbehandelte DNA mittels einer Polymerase und mindestens einem Primer amplifiziert wird, dessen 5'-Ende über einen Linker mit einer Sonde verbunden ist (Scorpion-Primer),
c) das Primer-Extensionsprodukt von dem Matrizenstrang getrennt wird,
d) die Sonde intramolekular an das Primer-Extensionsprodukt hybridisiert, wobei die Hybridisierung in Abhängigkeit von dem Methylierungsstatus der DNA erfolgt,
e) detektiert wird, ob eine Hybridisierung der Sonde stattgefunden hat.

2. Verfahren nach Anspruch 1, **dadurch gekennzeichnet, dass** die Umsetzung in Schritt a) mit einem Bisulfit erfolgt.

3. Verfahren nach Anspruch 1, **dadurch gekennzeichnet, dass** die Umsetzung in Schritt a) mittels einer Cytidin-Deaminase erfolgt, die unmethyliertes Cytidin schneller umsetzt als methyliertes Cytidin.

4. Verfahren nach mindestens einem der Ansprüche 1 bis 3, **dadurch gekennzeichnet, dass** die Amplifikation in Schritt b) mittels einer Polymerasekettenreaktion erfolgt.

5. Verfahren nach Anspruch 4, **dadurch gekennzeichnet, dass** die Polymerasekettenreaktion in Form des MSP- oder des Heavy Methyl-Verfahrens durchgeführt wird.

6. Verfahren nach mindestens einem der Ansprüche 1 bis 5, **dadurch gekennzeichnet, dass** die Sonde zwei Signalkomponenten trägt, die sich in der inaktiven Form in räumlicher Nähe zueinander befinden, und die durch die Hybridisierung der Sonde an ein Primer-Extensionsprodukt voneinander entfernt werden.

7. Verfahren nach Anspruch 6, **dadurch gekennzeichnet, dass** es sich bei den beiden Signalkomponenten um ein Quencher-Fluoreszenzfarbstoff-Paar handelt.

8. Verfahren nach mindestens einem der Ansprüche 6 bis 7, **dadurch gekennzeichnet, dass** die räumliche Trennung in der inaktiven Form durch eine Sekundärstruktur der Sonde, insbesondere durch eine Haarnadel, gewährleistet wird.

9. Verfahren nach mindestens einem der Ansprüche 1 bis 5, **dadurch gekennzeichnet, dass** der Scorpion-Primer zwei Signalkomponenten trägt, die in der inaktiven Form räumlich voneinander entfernt sind, und die durch die Hybridisierung der Sonde an ein Primer-Extensionsprodukt in räumliche Nähe zueinander gebracht werden.

10. Verfahren nach Anspruch 9, **dadurch gekennzeichnet, dass** die Signalkomponenten in der aktiven Form ein Signal über Fluoreszenz-Resonanz-Energie-Transfer erzeugen.

11. Verfahren nach mindestens einem der Ansprüche 1 bis 5, **dadurch gekennzeichnet, dass** die Sonde und ein weiteres Oligonukleotid jeweils mindestens eine Signalkomponente tragen, wobei sich die Signalkomponenten in der inaktiven Form in räumlicher Nähe zueinander befinden, und durch die Hybridisierung der Sonde an ein Primer-Extensionsprodukt voneinander entfernt werden.

12. Verfahren nach Anspruch 11, **dadurch gekennzeichnet, dass** es sich bei den beiden Signalkomponenten um ein Quencher-Fluoreszenzfarbstoff-Paar handelt.

13. Verfahren nach mindestens einem der Ansprüche 11 bis 12, **dadurch gekennzeichnet, dass** die räumliche Trennung in der inaktiven Form durch eine Duplexstruktur zwischen Sonde und dem weiteren Oligonukleotid gewährleistet wird.

14. Verfahren nach mindestens einem der Ansprüche 1 bis 5, **dadurch gekennzeichnet, dass** die Sonde und ein weiteres Oligonukleotid jeweils mindestens eine Signalkomponente tragen, wobei die Signalkomponenten in der inaktiven Form räumlich voneinander getrennt sind und durch die Hybridisierung der Sonde an ein Primer-Extensionsprodukt in räumliche Nähe zueinander gelangen.

15. Verfahren nach Anspruch 14, **dadurch gekennzeichnet, dass** die Signalkomponenten in der aktiven Form ein Signal über einen Fluoreszenz-Resonanz-Energie-Transfer erzeugen.

16. Verfahren nach mindestens einem der Ansprüche 14 bis 15, **dadurch gekennzeichnet, dass** das weitere Oligonukleotid in unmittelbarer Nähe zu der Sonde an das Primer-Extensionsprodukt bindet.

17. Verfahren nach mindestens einem der Ansprüche 1 bis 16, **dadurch gekennzeichnet, dass** mehrere Sequenzen gleichzeitig amplifiziert werden.

18. Verfahren nach mindestens einem der Ansprüche 1 bis 17, **dadurch gekennzeichnet, dass** die Amplifikation mittels zweier Scorpion-Primer erfolgt.

19. Verfahren nach Anspruch 18, **dadurch gekennzeichnet, dass** die Scorpion-Primer unterschiedliche Signalkomponenten tragen.

20. Verfahren nach mindestens einem der Ansprüche 18 bis 19, **dadurch gekennzeichnet, dass** einer der Scorpion-Primer eine methylierungsspezifische Sonde und der andere Scorpion-Primer eine nicht-methylierungsspezifische Sonde trägt.

21. Verfahren nach mindestens einem der Ansprüche 1 (bis 19, **dadurch gekennzeichnet, dass** einer der Scorpion-Primer eine methylierungsspezifische Sonde und der andere Scorpion-Primer eine mutations- oder allelspezifische Sonde trägt.

22. Verfahren nach mindestens einem der Ansprüche 1 bis 4, **dadurch gekennzeichnet, dass** eine nicht-methylierungsspezifische PCR-Amplifikation erfolgt, wobei die Sonde einen Quencher und ein Farbstoffmolekül trägt, die sich in der inaktiven Form in räumlicher Nähe zueinander befinden und die durch die Hybridisierung der Sonde an ein Primer-Extensionsprodukt voneinander entfernt werden ("Methyl-Hairpin").

23. Verfahren nach mindestens einem der Ansprüche 1 bis 4, **dadurch gekennzeichnet, dass** eine MSP-Amplifikation erfolgt, wobei die Sonde einen Quencher und ein Farbstoffmolekül trägt, die sich in der inaktiven Form in räumlicher Nähe zueinander befinden und die durch die Hybridisierung der Sonde an ein Primer-Extensionsprodukt voneinander entfernt werden ("MSP-Methyl-Hairpin").

24. Verfahren nach mindestens einem der Ansprüche 1 bis 4, **dadurch gekennzeichnet, dass** eine Heavy Methyl-Amplifikation erfolgt, wobei die Sonde einen Quencher und ein Farbstoffmolekül trägt, die sich in der inaktiven Form in räumlicher Nähe zueinander befinden und die bei Hybridisierung der Sonde an ein Primer-Extensionsprodukt voneinander entfernt werden ("Heavy Methyl-Hairpin").

25. Verfahren nach mindestens einem der Ansprüche 1 bis 4, **dadurch gekennzeichnet, dass** eine nicht-methylierungsspezifische Amplifikation erfolgt, wobei die Sonde ein Farbstoffmolekül und ein weiteres Oligonukleotid einen Quencher trägt, die sich in der inaktiven Form in räumlicher Nähe zueinander befinden und die durch die Hybridisierung der Sonde an ein Primer-Extensionsprodukt voneinander entfernt werden ("Methyl-Duplex").

26. Verfahren nach mindestens einem der Ansprüche 1 bis 4, **dadurch gekennzeichnet, dass** eine MSP-Amplifikation erfolgt, wobei die Sonde ein Farbstoffmolekül und ein weiteres Oligonukleotid einen Quencher trägt, die sich in der inaktiven Form in räumlicher Nähe zueinander befinden und die bei Hybridisierung der Sonde an ein Primer-Extensionsprodukt voneinander entfernt werden ("MSP-Methyl-Duplex").

27. Verfahren nach mindestens einem der Ansprüche 1 bis 4, **dadurch gekennzeichnet, dass** eine Heavy Methyl-Amplifikation erfolgt, wobei die Sonde ein Farbstoffmolekül und ein weiteres Oligonukleotid einen Quencher trägt, die sich in der inaktiven Form in räumlicher Nähe zueinander befinden und die bei Hybridisierung der Sonde an ein Primer-Extensionsprodukt voneinander entfernt werden ("Heavy Methyl-Duplex").

28. Verfahren nach Anspruch 23, **dadurch gekennzeichnet**, die Amplifikation mittels zweier Scorpion-Primer erfolgt, wobei einer der Scorpion-Primer eine methylierungsspezifische Sonde und der andere Scorpion-Primer eine nicht-methylierungsspezifische Sonde trägt ("quantitativer Methyl-Hairpin").

29. Verwendung der Verfahren nach mindestens einem der Ansprüche 1 bis 28 zur Diagnose oder Prognose von Krebserkrankungen oder anderen mit einer Veränderung des Cytosin-Methylierungsstatus assoziierten Krankheiten, zur Vorhersage von unerwünschten Arzneimittelwirkungen, zur Festlegung einer spezifischen Arzneimitteltherapie, zur Überwachung des Erfolges einer Arzneimitteltherapie, zur Unterscheidung von Zelltypen oder Geweben und zur Untersuchung der Zelldifferenzierung.

30. Verwendung von Scorpion-Primern zur Methylierungsanalyse von DNA aus Gewebeproben oder Körperflüssigkeiten, insbesondere zur Diagnose oder Prognose von Krebserkrankungen oder anderen mit einer Veränderung des Cytosin-Methylierungsstatus assoziierten Krankheiten, zur Vorhersage von unerwünschten Arzneimittelwirkungen, zur Festlegung einer spezifischen Arzneimitteltherapie, zur Überwachung des Erfolges einer Arzneimitteltherapie, zur Unterscheidung von Zelltypen oder Geweben und zur Untersuchung der Zelldifferenzierung.

31. Ein Kit zur Durchführung eines der Verfahren nach den Ansprüchen 1 bis 19, bestehend aus mindestens einem Scorpion-Primer, einer Polymerase, einem Bisulfitreagenz und den erforderlichen Reagenzien für eine Polymerasekettenreaktion.

## Claims

1. A method for the detection of cytosine methylations in DNA originated from tissue samples or body liquids is hereby **characterized in that**
a) the DNA to be investigated is reacted with a chemical or with an enzyme so that 5-methylcytosine remains unchanged, while unmethylated cytosine is converted to uracil or to another base which differs from cytosine in its base-pairing behavior,
b) the pretreated DNA is amplified by means of a polymerase and at least one primer, whose 5'-end is joined with a probe via a linker (Scorpion primer),
c) the primer extension product is separated from the matrix strand,
d) the probe hybridizes intramolecularly to the primer extension product, whereby the hybridization occurs as a function of the methylation state of the DNA,
e) a detection is made of whether a hybridization of the probe has occurred.

2. The method according to claim 1, further **characterized in that** the reaction in step a) is produced with a bisulfite.

3. The method according to claim 1, further **characterized in that** the reaction in step a) is produced by means of a cytidine deaminase; the unmethylated cytidine reacts more rapidly than methylated cytidine.

4. The method according to at least one of claims 1 to 3, further **characterized in that** the amplification in step b) is carried out by means of a polymerase chain reaction.

5. The method according to claim 4, further **characterized in that** the polymerase chain reaction is carried out in the form of the MSP or heavy methyl method.

6. The method according to at least one of claims 1 to 5, further **characterized in that** the probe bears two signal components which are found in spatial proximity to one another in the inactive form, and which are separated from one another by the hybridization of the probe to a primer extension product.

7. The method according to claim 6, further **characterized in that** the two signal components involve a quencher-fluorescent dye pair.

8. The method according to at least one of claims 6 to 7, further **characterized in that** the spatial separation in the inactive form is assured by the secondary structure of the probe, particularly by a hairpin shape.

9. The method according to at least one of claims 1 to 5, further **characterized in that** the Scorpion primer bears two signal components which are separated from one another in the inactive form, and which are brought into spatial proximity to one another by the hybridization of the probe to a primer extension product.

10. The method according to claim 9, further **characterized in that** the signal components in the active form generate a signal via fluorescence-resonance energy transfer.

11. The method according to at least one of claims 1 to 5, further **characterized in that** the probe and another oligonucleotide each bear at least one signal component, whereby the signal components are found in spatial proximity to one another in the inactive form, and are separated from one another by the hybridization of the probe to a primer extension product.

12. The method according to claim 11, further **characterized in that** the two signal components involve a quencher-fluorescent dye pair.

13. The method according to at least one of claims 11 to 12, further **characterized in that** the spatial separation between the probe and the other oligonucleotide in the inactive form is assured by a duplex structure.

14. The method according to at least one of claims 1 to 5, further **characterized in that** the probe and another oligonucleotide each bear at least one signal component, whereby the signal components are separated from one another spatially in the inactive form, and are brought into spatial proximity to one another by the hybridization of the probe to a primer extension product.

15. The method according to claim 14, further **characterized in that** the signal components in the active form generate a signal via a fluorescence-resonance energy transfer.

16. The method according to at least one of claims 14 to 15, further **characterized in that** the other oligonucleotide binds in immediate proximity to the probe on the primer extension product.

17. The method according to at least one of claims 1 to 16, further **characterized in that** several sequences are simultaneously amplified.

18. The method according to at least one of claims 1 to 17, further **characterized in that** the amplification occurs by means of two Scorpion primers.

19. The method according to claim 18, further **characterized in that** the Scorpion primers bear different signal components.

20. The method according to at least one of claims 18 to 19, further **characterized in that** one of the Scorpion primers bears a methylation-specific probe and the other Scorpion primer bears a non-methylation-specific probe.

21. The method according to at least one of claims 1 (to 19, further **characterized in that** one of the Scorpion primers bears a methylation-specific probe and the other Scorpion primer bears a mutation-specific or allele-specific probe.

22. The method according to at least one of claims 1 to 4, further **characterized in that** a non-methylation-specific PCR amplification takes place, wherein the probe bears a quencher and a dye molecule, which are found in spatial proximity to one another in the inactive form, and which are separated from one another by the hybridization of the probe to a primer extension product ("methyl hairpin").

23. The method according to at least one of claims 1 to 4, further **characterized in that** an MSP amplification takes place, wherein the probe bears a quencher and a dye molecule which are found in spatial proximity to one another in the inactive form, and which are separated from one another by the hybridization of the probe to a primer extension product ("MSP methyl hairpin").

24. The method according to at least one of claims 1 to 4, further **characterized in that** a heavy methyl amplification takes place, wherein the probe bears a quencher and a dye molecule which are found in spatial proximity to one another in the inactive form, and which are separated from one another in the hybridization of the probe to a primer extension product ("heavy methyl hairpin").

25. The method according to at least one of claims 1 to 4, further **characterized in that** a non-methylation-specific amplification takes place, wherein the probe bears a dye molecule and another oligonucleotide bears a quencher which are found in spatial proximity to one another in the inactive form, and which are separated from one another by the hybridization of the probe to a primer extension product ("methyl duplex").

26. The method according to at least one of claims 1 to 4, further **characterized in that** an MSP amplification takes place, wherein the probe bears a dye molecule and another oligonucleotide bears a quencher which are found in spatial proximity to one another in the inactive form, and which are separated from one another in the hybridization of the probe to a primer extension product ("MSP methyl duplex").

27. The method according to at least one of claims 1 to 4, further **characterized in that** a heavy methyl amplification takes place, wherein the probe bears a dye molecule and another oligonucleotide bears a quencher which are found in spatial proximity to one another in the inactive form, and which are separated from one another in the hybridization of the probe to a primer extension product ("heavy methyl duplex").

28. The method according to claim 23, further **characterized in that** the amplification is produced by means of two Scorpion primers, wherein one of the Scorpion primers bears a methylation-specific probe and the other Scorpion primer bears a non-methylation-specific probe ("quantitative methyl hairpin").

29. Use of the method according to at least one of claims 1-28 for the diagnosis or prognosis of cancer disorders or other diseases associated with a change in the cytosine methylation state, for predicting undesired drug interactions, for establishing a specific drug therapy, for monitoring the success of a drug therapy, for the differentiation of cell types or tissues and for the investigation of cell differentiation.

30. Use of Scorpion primers for methylation analysis in DNA originated from tissue samples or body liquids, particularly for the diagnosis or prognosis of cancer disorders or other diseases associated with a change in the cytosine methylation state, for predicting undesired drug interactions, for establishing a specific drug therapy, for monitoring the success of a drug therapy, for the differentiation of cell types or tissues and for the investigation of cell differentiation.

31. A kit for performing a method according to claims 1 to 19, consisting of at least one Scorpion primer, a polymerase, a bisulfite reagent and the necessary reagents for a polymerase chain reaction

## Revendications

1. Procédé pour détecter des méthylations de la cytosine dans de l'ADN provenant d'échantillons tissulaires ou de liquides corporels, **caractérisé en ce que**
a) on fait réagir l'ADN devant être étudié à l'aide d'un produit chimique ou d'une enzyme de telle sorte que la 5-méthylcytosine reste inchangée, tandis que la cytosine non méthylée est convertie en uracile ou en une autre base, qui se distingue de la cytosine par son comportement en appariement des bases,
b) on amplifie l'ADN prétraité avec une polymérase et au moins une amorce, dont l'extrémité 5' est liée à une sonde par l'intermédiaire d'un lieur (amorce Scorpion),
c) on sépare du brin-matrice le produit d'extension d'amorce,
d) on hybride la sonde, d'une manière intramoléculaire, au produit d'extension d'amorce, l'hybridation étant effectuée en fonction de l'état de méthylation de l'ADN,
e) on détecte si une hybridation de la sonde a eu lieu.

2. Procédé selon la revendication 1, **caractérisé en ce que** la réaction de l'étape a) est effectuée avec un bisulfite.

3. Procédé selon la revendication 1, **caractérisé en ce que** la réaction de l'étape a) est réalisée à l'aide d'une cytidine-désaminase, qui fait réagir la cytidine non méthylée plus vite que la cytidine méthylée.

4. Procédé selon au moins l'une des revendications 1 à 3, **caractérisé en ce que** l'amplification de l'étape b) est réalisée à l'aide d'une réaction en chaîne par polymérase.

5. Procédé selon la revendication 4, **caractérisé en ce que** la réaction en chaîne par polymérase est mise en oeuvre sous forme du procédé MSP ou du procédé Heavy Methyl.

6. Procédé selon au moins l'une des revendications 1 à 5, **caractérisé en ce que** la sonde porte deux composants de signal, qui sous la forme inactive sont spatialement proches l'un de l'autre, et qui s'éloignent l'un de l'autre sous l'effet de l'hybridation de la sonde à un produit d'extension d'amorce.

7. Procédé selon la revendication 6, **caractérisé en ce que**, pour ce qui concerne les deux composants de signal, il s'agit d'une paire fluorophore receveur (quencher)-colorant fluorescent.

8. Procédé selon au moins l'une des revendications 6 à 7, **caractérisé en ce que** la séparation spatiale sous la forme inactive est assurée par une structure secondaire de la sonde, en particulier par une boucle en épingle à cheveux.

9. Procédé selon au moins l'une des revendications 1 à 5, **caractérisé en ce que** l'amorce Scorpion porte deux composants de signal, qui sous la forme inactive sont spatialement éloignés l'un de l'autre, et qui sont spatialement rapprochés l'un de l'autre sous l'effet de l'hybridation de la sonde à un produit d'extension d'amorce.

10. Procédé selon la revendication 9, **caractérisé en ce que** les composants de signal sous la forme active produisent un signal sous l'effet d'un transfert d'énergie de résonance de fluorescence.

11. Procédé selon au moins l'une des revendications 1 à 5, **caractérisé en ce que** la sonde et un autre oligonucléotide portent chacun au moins un composant de signal, les composants de signal sous la forme inactive étant spatialement proches l'un de l'autre, et étant éloignés l'un de l'autre sous l'effet de l'hybridation de la sonde à un produit d'extension d'amorce.

12. Procédé selon la revendication 11, **caractérisé en ce que** les deux composants de signal sont une paire fluorophore receveur (quencher)-colorant fluorescent.

13. Procédé selon au moins l'une des revendications 11 à 12, **caractérisé en ce que** la séparation spatiale sous la forme inactive est assurée par une structure duplex entre la sonde et l'autre oligonucléotide.

14. Procédé selon au moins l'une des revendications 1 à 5, **caractérisé en ce que** la sonde et un autre oligonucléotide portent chacun au moins un composant de signal, les composants de signal sous la forme inactive étant spatialement séparés l'un de l'autre, et étant spatialement rapprochés l'un de l'autre sous l'effet de l'hybridation de la sonde à un produit d'extension d'amorce.

15. Procédé selon la revendication 14, **caractérisé en ce que** les composants de signal sous la forme active produisent un signal sous l'effet d'un transfert d'énergie de résonance par fluorescence.

16. Procédé selon au moins l'une des revendications 14 à 15, **caractérisé en ce que** l'autre oligonucléotide se lie au produit d'extension d'amorce à proximité immédiate de la sonde.

17. Procédé selon au moins l'une des revendications 1 à 16, **caractérisé en ce que** plusieurs séquences subissent simultanément une amplification.

18. Procédé selon au moins l'une des revendications 1 à 17, **caractérisé en ce que** l'amplification est effectuée à l'aide de deux amorces Scorpion.

19. Procédé selon la revendication 18, **caractérisé en ce que** les amorces Scorpion portent différents composants de signal.

20. Procédé selon au moins l'une des revendications 18 à 19, **caractérisé en ce que** l'une des amorces Scorpion porte une sonde spécifique de méthylation, et l'autre amorce Scorpion porte une sonde non spécifique de méthylation.

21. Procédé selon au moins l'une des revendications 1 (à 19, **caractérisé en ce que** l'une des amorces Scorpion porte une sonde spécifique de méthylation, et l'autre amorce Scorpion porte une sonde spécifique de mutation ou d'allèle.

22. Procédé selon au moins l'une des revendications 1 à 4, **caractérisé en ce qu'**on procède à une amplification PCR non spécifique de méthylation, la sonde portant un fluorophore receveur (quencher) et une molécule de colorant, qui sous la forme inactive se trouvent spatialement près l'un de l'autre, et qui sont séparés l'un de l'autre sous l'effet de l'hybridation de la sonde à un produit d'extension d'amorce ("Methyl-Hairpin").

23. Procédé selon au moins l'une des revendications 1 à 4, **caractérisé en ce qu'**on procède à une amplification MSP, la sonde portant un fluorophore receveur (quencher) et une molécule de colorant, qui sous la forme inactive sont spatialement proches l'un de l'autre, et qui sont éloignés l'un de l'autre sous l'effet de l'hybridation de la sonde à un produit d'extension d'amorce ("MSP-Methyl-Hairpin").

24. Procédé selon au moins l'une des revendications 1 à 4, **caractérisé en ce qu'**on procède à une amplification Heavy Methyl, la sonde portant un fluorophore receveur (quencher) et une molécule de colorant, qui sous la forme inactive sont spatialement proches l'un de l'autre, et qui sont éloignés l'un de l'autre lors de l'hybridation de la sonde à un produit d'extension d'amorce ("Heavy Methyl-Hairpin").

25. Procédé selon au moins l'une des revendications 1 à 4, **caractérisé en ce qu'**on procède à une amplification non spécifique de méthylation, la sonde portant une molécule de colorant, et un autre oligonucléotide portant un fluorophore receveur (quencher), qui sous la forme inactive sont spatialement proches l'un de l'autre, et qui sont éloignés l'un de l'autre sous l'effet de l'hybridation de la sonde à un produit d'extension d'amorce ("Methyl-Duplex").

26. Procédé selon au moins l'une des revendications 1 à 4, **caractérisé en ce qu'**on procède à une amplification MSP, la sonde portant une molécule de colorant, et un autre oligonucléotide portant un fluorophore receveur (quencher), qui sous la forme inactive sont spatialement proches l'un de l'autre, et qui sont éloignés l'un de l'autre lors de l'hybridation de la sonde à un produit d'extension d'amorce ("MSP-Methyl-Duplex").

27. Procédé selon au moins l'une des revendications 1 à 4, **caractérisé en ce qu'**on procède à une amplification Heavy Methyl, la sonde portant une molécule de colorant, et un autre oligonucléotide portant un fluorophore receveur (quencher), qui sous la forme inactive sont spatialement proches l'un de l'autre, et qui sont éloignés l'un de l'autre lors de l'hybridation de la sonde à un produit d'extension d'amorce ("Heavy Methyl-Duplex").

28. Procédé selon la revendication 23, **caractérisé en ce qu'**on procède à l'amplification à l'aide de deux amorces Scorpion, l'une des amorces Scorpion portant une sonde spécifique de méthylation, et l'autre amorce Scorpion portant une sonde non spécifique de méthylation ("Methyl-Hairpin quantitatif").

29. Utilisation des procédés selon au moins l'une des revendications 1 à 28 pour le diagnostic ou le pronostic de maladies cancéreuses ou d'autres maladies associées à une modification de l'état de méthylation de la cytosine, pour prédire des effets médicamenteux indésirables, pour définir un traitement médicamenteux spécifique, pour surveiller le succès d'un traitement médicamenteux, pour distinguer des types cellulaires ou des tissus, et pour étudier la différenciation cellulaire.

30. Utilisation d'amorces Scorpion pour l'analyse de la méthylation de l'ADN provenant d'échantillons tissulaires ou de fluides corporels, en particulier pour le diagnostic ou le pronostic de maladies cancéreuses ou d'autres maladies associées à une modification de l'état de méthylation de la cytosine, pour prédire des effets médicamenteux indésirables, pour définir un traitement médicamenteux spécifique, pour surveiller le succès d'un traitement médicamenteux, pour distinguer des types cellulaires ou des tissus, et pour étudier la différenciation cellulaire.

31. Trousse pour mettre en oeuvre l'un des procédés selon les revendications 1 à 19, constituée d'au moins une amorce Scorpion, d'une polymérase, d'un réactif de type bisulfite, et des réactifs nécessaires à une réaction en chaîne par polymérase.
